# EUROPEAN PATENT APPLICATION

(11) **EP 1 939 191 A1**
(43) Date of publication of application: **02.07.2008**
(21) Application number: 06127276.1
(22) Date of filing: 28.12.2006
(51) Int. Cl.: C07D 307/42, C07D 307/54, A61K 31/341, A61P 25/28

(54) **Furan derivatives, method of synthesis and uses thereof**

(71) Applicant: Neuropharma S.A., 28760 Tres Cantos (Madrid) (ES)
(72) Inventor: Martinez Gil, Ana, 28004 Madrid (ES); Medina Padilla, Miguel, 28035 Madrid (ES); Fall Diop, Yagamare, 36208 Vigo, Pontevedra (ES); Gomez Pacios, Generosa, 36208 Vigo, Pontevedra (ES); Pérez Vazquez, Manuel, 36208 Vigo, Pontevedra (ES); Martin Aparicio, Ester, 28411 Moralzarzal, Madrid (ES); Fuertes Huerta, Ana, 28003 Madrid (ES); Del Monte Millan, Maria, 28035 Madrid (ES); Navarro Rico, Maria Luisa, 28014 Madrid (ES); Pérez Puerto, Maria, José, 28008 Madrid (ES)
(74) Representative: ABG Patentes, S.L.

(57) **Abstract**

The present invention relates to furan derivatives of formula (I), their method of synthesis and uses thereof. Concretely, the compounds disclosed have proved to be inhibitors of glycogen synthase kinase 3β, GSK-3 β, which is known to be involved in different disease and conditions, such as Alzheimer's disease or non-insulin dependent diabetes mellitus. The present invention also relates to pharmaceutical compositions comprising the same. Further, the present invention is directed to the use of the compounds in the manufacture of a medicament for the treatment and/or prevention of a GSK-3 mediated disease or condition.

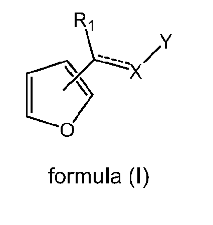

## Description

### FIELD OF THE INVENTION

The present invention relates to furan derivatives, their method of synthesis and uses thereof. Concretely, the compounds disclosed have proved to be inhibitors of glycogen synthase kinase 3β, GSK-3 β, which is known to be involved in different diseases and conditions, such as Alzheimer's disease or non-insulin dependent diabetes mellitus. The present invention also relates to pharmaceutical compositions comprising the same. Further, the present invention is directed to the use of the compounds in the manufacture of a medicament for the treatment and/or prevention of a GSK-3 mediated disease or condition.

### BACKGROUND OF THE INVENTION

The search for new therapeutic agents has been greatly aided in recent years by a better understanding of the structure of enzymes and other biomolecules associated with target diseases. One important class of enzymes that has been the subject of extensive study are the protein kinases. Many diseases are associated with abnormal cellular responses triggered by protein kinase-mediated events. These diseases include autoimmune diseases, inflammatory diseases, neurological and neurodegenerative diseases, cancer, cardiovascular diseases, allergies and asthma, Alzheimer's disease or hormone-related diseases. Accordingly, there has been a substantial effort in medicinal chemistry to find protein kinase inhibitors that are effective as therapeutic agents.

Glycogen synthase kinase-3 (GSK-3) is a serine/threonine protein kinase comprised of α and β isoforms that are each encoded by distinct genes (Coghlan et al., Chemistry & Biology, 7, 793-803 (2000); Kim and Kimmel, Curr. Opinion Genetics Dev., 10, 508-514 (2000)). The threonine/serine kinase glycogen synthase kinase-3 (GSK-3) fulfills a pivotal role in various receptor-linked signalling pathways (Doble, BW, Woodgett, JR J.Cell Sci. 2003, 116:1175-1186). Dysregulation within these pathways is considered a crucial event in the development of several prevalent human disorders, such as type (II) diabetes (Kaidanovich O, Eldar-Finkelman H, Expert Opin. Ther. Targets, 2002, 6:555-561), Alzheimer's disease (Grimes CA, Jope RS, Prog.Neurobiol. 2001, 65:391-426), CNS disorders such as manic depressive disorder and neurodegenerative diseases, and chronic inflammatory disorders (Hoeflich KP, Luo J, Rubie EA, Tsao MS, Jin O, Woodgett J, Nature 2000, 406:86-90). These diseases may be caused by, or result in, the abnormal operation of certain cell signalling pathways in which GSK-3 plays a role.

GSK-3 has been found to phosphorylate and modulate the activity of a number of regulatory proteins. These proteins include glycogen synthase which is the rate limiting enzyme necessary for glycogen synthesis, the microtubule associated protein Tau, the gene transcription factor β-catenin, the translation initiation factor e1F2B, as well as ATP citrate lyase, axin, heat shock factor-1, c-Jun, c-Myc, c-Myb, CREB, and CEPBα. These diverse protein targets implicate GSK-3 in many aspects of cellular metabolism, proliferation, differentiation and development.

Currently, inhibition of GSK-3 may represent a viable strategy to develop novel medicinal entities for the treatment of such unmet diseases (Martinez A, Castro A, Dorronsoro I, Alonso M, Med. Res. Rev., 2002, 22:373-384) through insulin mimicry, tau dephosphorylation and amyloid processing, or transcriptional modulation respectively.

There is still a need to find good GSK-3 inhibitors, being both effective and selective, and having good "drugability" properties, i.e. good pharmaceutical properties related to administration, distribution, metabolism and excretion. An additional advantage would be to find compounds with simple, stable structures, being easy to prepare by ordinary proceedings known to the skilled person.

### SUMMARY OF THE INVENTION

It has now been found that a group of stable and small furan derivatives shows inhibitory effects on GSK-3 enzyme.

Thus, according to a first aspect, the present invention is directed to a compound of formula (I) 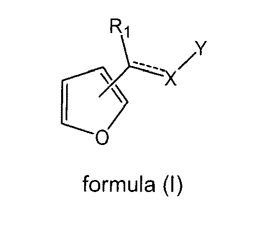 wherein
X is selected from the group consisting of -O-, -S-, -N(R^{d})-, -CH(R^{c})- and - C(R^{c})-; wherein
   R^{c} is selected from the group consisting of hydrogen, substituted or unsubstituted C₁-C₃ alkyl, substituted or unsubstituted C₂-C₄ alkenyl, substituted or unsubstituted aryl, and substituted or unsubstituted aralkyl;
   R^{d} is selected from the group consisting of hydrogen, substituted or unsubstituted alkyl, substituted or unsubstituted alkenyl, substituted or unsubstituted aryl, substituted or unsubstituted aralkyl, substituted or unsubstituted cycloalkyl, and substituted or unsubstituted heterocyclyl;
R₁ is selected from the group consisting of -H and -OH;
when X is -C(R^{c})-, the dotted line represents an additional bond; and
Y is selected from the group consisting of
   -(CH₂)ₚW, wherein p is an integer selected from 0, 1, 2, 3, 4, 5 and 6 and W is selected from the group consisting of -OR^{a}, -SR^{a} and -NR^{a}R^{b}; and
   -(CH₂)ₚ-Z-(CH₂)_{q}-C(R₂)(R₃)W, wherein p and W are as defined above; q is an integer selected from 0, 1, 2, 3 and 4; and Z is selected from the group consisting of unsubstituted alkyl, unsubstituted alkenyl, alkyl substituted with at least one C₁-C₃ alkyl group and alkenyl substituted with at least one C₁-C₃ alkyl group; and R₂ and R₃ are both hydrogen or together are =O;
      wherein R^{a} and R^{b} are independently selected from the group consisting of hydrogen, protecting group, substituted or unsubstituted alkyl, substituted or unsubstituted cycloalkyl, substituted or unsubstituted alkenyl, substituted or unsubstituted aryl, substituted or unsubstituted heterocyclyl and substituted or unsubstituted aralkyl;
with the proviso that if R₁ is -OH, X is -CH(R^{c})-, R^{c} being hydrogen, Y is -(CH₂)ₚW, p being 0 and W being -OR^{a}, then R^{a} is not hydrogen;
its salts or solvates thereof.

According to a further aspect, the present invention is directed to a compound of formula (I) as defined above, its salts or solvates thereof, for use as a medicament.

According to a further aspect, the present invention is directed to the use of a compound of formula (I) as defined above, its salts or solvates thereof, in the manufacture of a medicament for the treatment and/or prevention of a GSK-3 mediated disease or condition.

According to a further aspect, the present invention is directed to a pharmaceutical composition comprising at least one compound of formula (I) as defined above, its salts or solvates thereof, and at least one pharmaceutically acceptable carrier.

According to a further aspect, the present invention is directed to the use of a compound of formula (I) as defined above, its salts or solvates thereof, as reagent for biological assays, preferably as a reagent for GSK-3 inhibition.

According to a further aspect, the present invention is directed to a method of treating or preventing a GSK-3 mediated disease or condition, which method comprises administering to a patient in need of such a treatment a therapeutically effective amount of at least one compound of formula (I) as defined as defined above, its salts or solvates thereof, or a pharmaceutical composition thereof.

According to a further aspect, the present invention is directed to a process for the synthesis of the compounds of formula (I), its salts or solvates thereof.

### DETAILED DESCRIPTION OF THE INVENTION

### Compounds of formula (I)

As mentioned above, an aspect of the present invention is a compound of formula (I), its salts or solvates thereof.

The compounds of formula (I) may be in the form of salts, preferably pharmaceutically acceptable salts, or in the form of solvates. The term **"pharmaceutically acceptable salts"** refers to any salt which upon administration to the recipient is capable of providing (directly or indirectly) a compound as described herein. However, it will be appreciated that non-pharmaceutically acceptable salts also fall within the scope of the invention since those may be useful in the preparation of pharmaceutically acceptable salts. Preferably, "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

The term "**solvate**" according to this invention is to be understood as meaning any form of the active compound according to the invention which has another molecule (most likely a polar solvent) attached to it via non-covalent bonding. Examples of solvates include hydrates and alcoholates, e.g. methanolate. Preferably, the solvates are pharmaceutically acceptable solvates.

The preparation of salts and solvates can be carried out by methods known in the art. For instance, pharmaceutically acceptable salts of compounds provided herein are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium, ammonium, magnesium, aluminium and lithium salts, and organic alkali salts such as, for example, ethylenediamine, ethanolamine, N,N-dialkylenethanolamine, triethanolamine, glucamine and basic aminoacids salts.

One preferred pharmaceutically acceptable form is the crystalline form, including such form in a pharmaceutical composition. In the case of salts and solvates the additional ionic and solvent moieties must also be non-toxic. The compounds of the invention may present different **polymorphic forms**, it is intended that the invention encompasses all such forms.
According to an embodiment of the invention, the compound of formula (I) is a compound of formula (II) 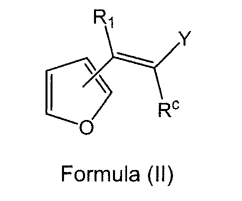 wherein R^{c}, R₁ and Y are as defined in formula (I);
its salts or solvates thereof.
According to an embodiment of the invention, the compound of formula (I) is a compound of formula (III) 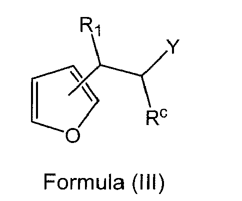 wherein R^{c}, R₁ and Y are as defined in formula (I);
its salts or solvates thereof.

In the compounds of formula (I), (II) or (III), R₁ is preferably hydrogen.

According to an embodiment of the invention, the compound of formula (I) is a compound of formula (IV) 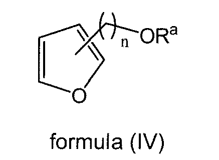 wherein R^{a} is as defined in formula (I) and n is 2, 3, 4, 5, 6, 7 or 8; its salts or solvates thereof.

According to an embodiment of the invention, the compound of formula (I) is a compound of formula (V) 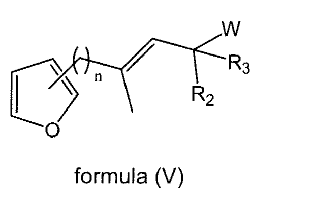 wherein W, R₂ and R₃ are as defined in formula (I) and n is 2, 3, 4, 5, 6, 7 or 8;
its salts or solvates thereof.

According to an embodiment of the invention, the compound of formula (I) is a compound of formula (VII) 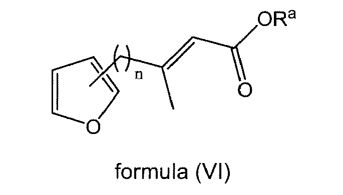 wherein R^{a} is as defined in formula (I) and n is 2, 3, 4, 5, 6, 7 or 8;
its salts or solvates thereof.
According to an embodiment of the invention, the compound of formula (I) is a compound of formula (VI) 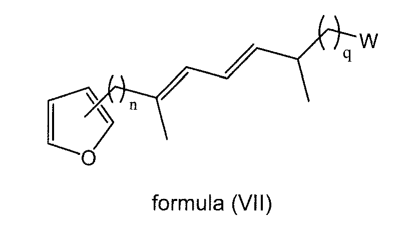 wherein W is as defined in formula (I);
n is 2, 3, 4, 5, 6, 7 or 8; and
q is 2, 3 or 4; its salts or solvates thereof.

According to a preferred embodiment, the compound of formula (VII) is a compound wherein W is -ORa.

According to an embodiment of the invention, the compound of formula (I) is a compound of formula (VIII) 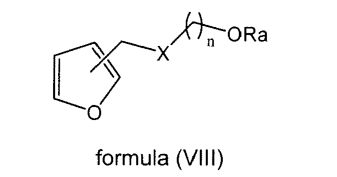 wherein X and R^{a} are as defined in formula (I), and n is 2, 3, 4, 5, 6, 7 or 8;
its salts or solvates thereof.
According to a preferred embodiment, X is -S- or -O- for the compounds of formula (VIII).
According to an embodiment of the invention, the compound of formula I is a compound of formula IX 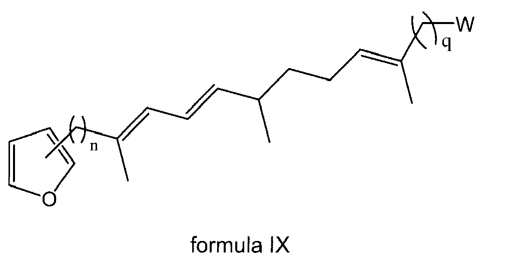 wherein W is as defined in formula (I);
n is 2, 3, 4, 5, 6, 7 or 8; and
q is 2, 3 or 4; its salts or solvates thereof.

According to a preferred embodiment, the compound of formula (I) is selected from the group consisting of
- *tert*-Butyl-(2-furan-2-yl-ethoxy)-diphenyl-silane;
- 2-(*tert*-Butyl-diphenyl-silanyloxy)-1-furan-2-yl-ethanol;
- *tert*-Butyl-(3-furan-2-yl-propoxy)-diphenyl-silane;
- *tert*-Butyl-(5-furan-2-yl-pentyloxy)-diphenyl-silane;
- *tert*-Butyl-(6-furan-2-yl-hexyloxy)-diphenyl-silane;
- *tert*-Butyl-[3-(furan-2-ylmethylsulfanyl)-propoxy]-diphenyl-silane
- 6-Furan-3-yl-3-methyl-hex-2-enoic acid ethyl ester;
- 3-Furan-3-yl-prop-2-en-1-ol;
- *tert*-Butyl-(11-furan-3-yl-4,8-dimethyl-undeca-5,7-dienyloxy)-diphenyl-silane;
- 2,2-Dimethyl-propionic acid 13-furan-3-yl-2,6,10-trimethyl-trideca-2,7,9-trienyl ester; and
- 13-Furan-3-yl-2,6,10-trimethyl-trideca-2,7,9-trien-1-ol;
its salts or solvates thereof.

### Uses of compounds of formula (I) and pharmaceutical compositions

As mentioned above, a further aspect of the present invention is the use of a compound of formula (I), its salts or solvates thereof; in the manufacture of a medicament for the treatment and/or prevention of a GSK-3 mediated disease or condition.

Within the present invention, the expression "GSK-3 mediated disease or condition" means any disease or other deleterious condition or state in which GSK-3 is known to play a role. According to a preferred embodiment, said GSK-3 mediated disease or condition is selected from diabetes, conditions associated with diabetes, chronic neurodegenerative conditions including dementias such as Alzheimer's Disease, Parkinson's disease, progressive supranuclear palsy, subacute sclerosing panencephalitic parkinsonism, postencephalitic parkinsonism, pugilistic encephalitis, guam parkinsonism-dementia complex, Pick's disease, corticobasal degeneration, frontotemporal dementia, Huntington's Disease, AIDS associated dementia, amyotrophic lateral sclerosis, multiple sclerosis and neurotraumatic diseases such as acute stroke, epilepsy, mood disorders such as depression, schizophrenia and bipolar disorders, promotion of functional recovery post stroke, cerebral bleeding such as due to solitary cerebral amyloid angiopathy, hair loss, obesity, atherosclerotic cardiovascular disease, hypertension, polycystic ovary syndrome, syndrome X, ischaemia, brain injury, traumatic brain injury, cancer, leukopenia, Down's syndrome, Lewy body disease, inflammation, chronic inflammatory diseases, cancer and hyperproliferative diseases as hyperplasias and immunodeficiency.

According to a preferred embodiment, said GSK-3 mediated disease or condition is Alzheimer's Disease.

According to a preferred embodiment, said GSK-3 mediated disease or condition is type (II) diabetes.

According to a preferred embodiment, said GSK-3 mediated disease or condition is depression.

According to a preferred embodiment, said GSK-3 mediated disease or condition is brain injury.

According to a further aspect, the present invention is directed to a compound of formula (I), its salts or solvates thereof, as defined above for use as a medicament.

According to a further aspect, the present invention is directed to the use of a compound of formula (I), its salts or solvates thereof, as defined above as reagents for biological assays, preferably as a reactive for GSK-3 inhibition. Said method comprises contacting a biological sample with a GSK-3 inhibitor of formula (I). The term **"biological sample",** as used herein, includes, without limitation, cell cultures or extracts thereof; preparations of an enzyme suitable for in vitro assay; biopsied material obtained from a mammal or extracts thereof; and blood, saliva, urine, feces, semen, tears, or other body fluids or extracts thereof. Thus, in one aspect the invention is directed to the use of compounds of formula (I) as reagents for biological assays, in particular as a reagent for GSK-3 inhibition.

According to a further aspect, the present invention is directed to a method of treating or preventing a GSK-3 mediated disease or condition, which method comprises administering to a patient in need of such a treatment a therapeutically effective amount of at least one compound of formula (I), its salts or solvates thereof, as defined above or a pharmaceutical composition thereof.

According to a further aspect, the present invention is directed to a pharmaceutical composition comprising at least one compound of formula (I) as defined above, its salts or solvates thereof, and at least one pharmaceutically acceptable carrier.

The term "**carrier**" refers to a **diluent, adjuvant, excipient, or vehicle** with which the active ingredient is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E.W. Martin, 1995.

Preferably, the carriers of the invention are approved by a regulatory agency of the Federal or a state government or listed in the U.S. Pharmacopeia or other generally recognized pharmacopeia for use in animals, and more particularly in humans

The carriers and auxilliary substances necessary to manufacture the desired pharmaceutical form of administration of the pharmaceutical composition of the invention will depend, among other factors, on the elected administration pharmaceutical form. Said pharmaceutical forms of administration of the pharmaceutical composition will be manufactured according to conventional methods known by the skilled person in the art. A review of different active ingredient administration methods, excipients to be used and processes for producing them can be found in "Tratado de Farmacia Galénica", C. Faulí i Trillo, Luzán 5, S.A. de Ediciones, 1993.

Examples of pharmaceutical compositions include any solid (tablets, pills, capsules, granules etc.) or liquid (solutions, suspensions or emulsions) composition for oral, topical or parenteral administration.

In a preferred embodiment the pharmaceutical compositions are in oral form. Suitable dose forms for oral administration may be tablets and capsules and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycolate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the appropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Administration of the compounds or compositions of the present invention may be by any suitable method, such as intravenous infusion, oral preparations, and intraperitoneal and intravenous administration. Oral administration is preferred because of the convenience for the patient and the chronic character of many of the diseases to be treated.

Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

The compounds and compositions of this invention may be used with other drugs to provide a combination therapy. The other drugs may form part of the same composition, or be provided as a separate composition for administration at the same time or at different time.

### Process for the synthesis of a compound of formula (I)

The compounds of the present invention may be prepared by the methods described below.

For example, a starting material for the synthesis of a compound of formula (I) may be furan. Alkylation of furan with an alkyl halide of formula (XI) 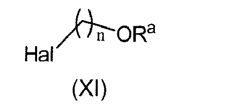 wherein n and R^{a} are as defined in formula (IV), provides a 2-substituted furan of formula (IV) (scheme 1):

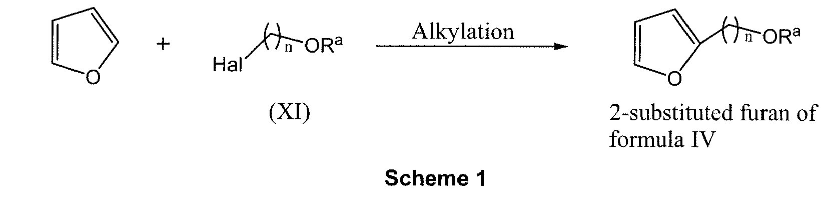

Compounds of formula (II), (III), (V), (VI), (VII) or (IX) may be obtained by means of a convergent strategy comprising two fragments:
a) a first fragment being synthesized through a sequence of successive chain elongations starting from compounds of formula (XIV) 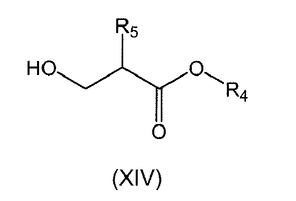 wherein the -C(=O)OR₄ fragment forms an ester group, R₄ being preferably selected from the group consisting of substituted or unsubstituted alkyl, substituted or unsubstituted alkenyl, substituted or unsubstituted aryl, substituted or unsubstituted aralkyl, substituted or unsubstituted cycloalkyl, and substituted or unsubstituted heterocyclyl; and R₅ is selected from the group consisting of hydrogen, unsubstituted alkyl, unsubstituted alkenyl, alkyl substituted with at least one C₁-C₃ alkyl group and alkenyl substituted with at least one C₁-C₃ alkyl group; and
b) a second fragment may be obtained by a sequence of chain elongations starting from 3-furancarboxaldehyde or 2-furancarboxaldehyde;

Once fragments a) and b) have been prepared they may be coupled.

For example, a chain elongation in order to obtain fragment a) is shown in scheme 2:

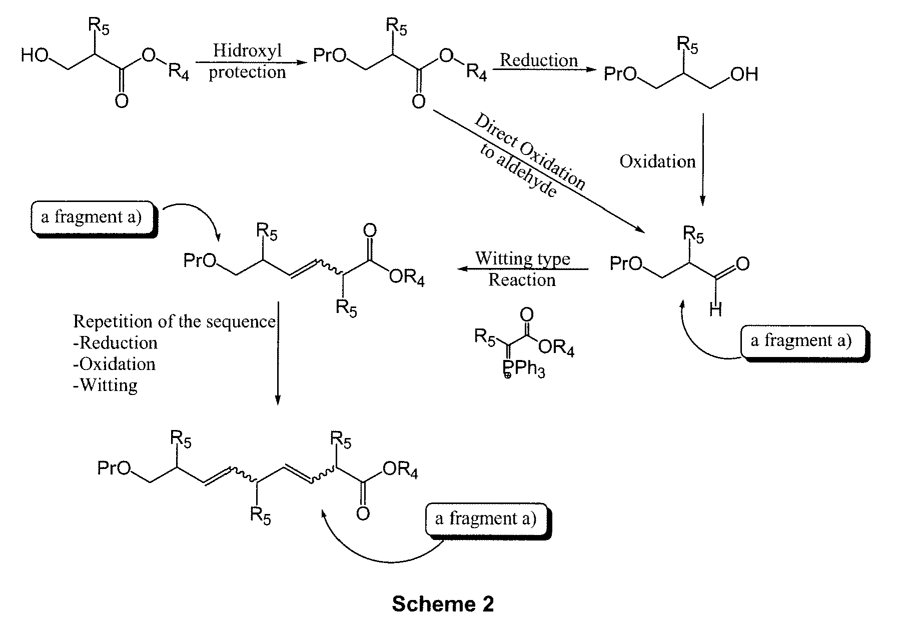

Hydroxyl protection can be performed by methods known to the skilled person such as those described in Greene and Wuts' "Protective Groups in Organic Synthesis", John Wiley & Sons, Inc., New York, 4th Ed., 2007. The ester moiety may be reduced to a hydroxyl group by common hydride reagents, such as Lithium aluminium hydride, and then oxidized to the corresponding aldehyde by means of mild oxidants such as PCC or NMO/TPAP. The conditions for Witting type reactions are well known in the art. For example see Maryanoff; Reitz, Chem. Rev. 1989, 89, 863-927 or March, "Advanced Organic Chemistry: Reactions, Mechanisms, and Structure", John Wiley & Sons, Inc., New York, 4th Ed., 1992.

An alternative chain elongation in order to obtain fragment a) is shown in scheme 3 which comprises halogenation, alkylation and reduction.

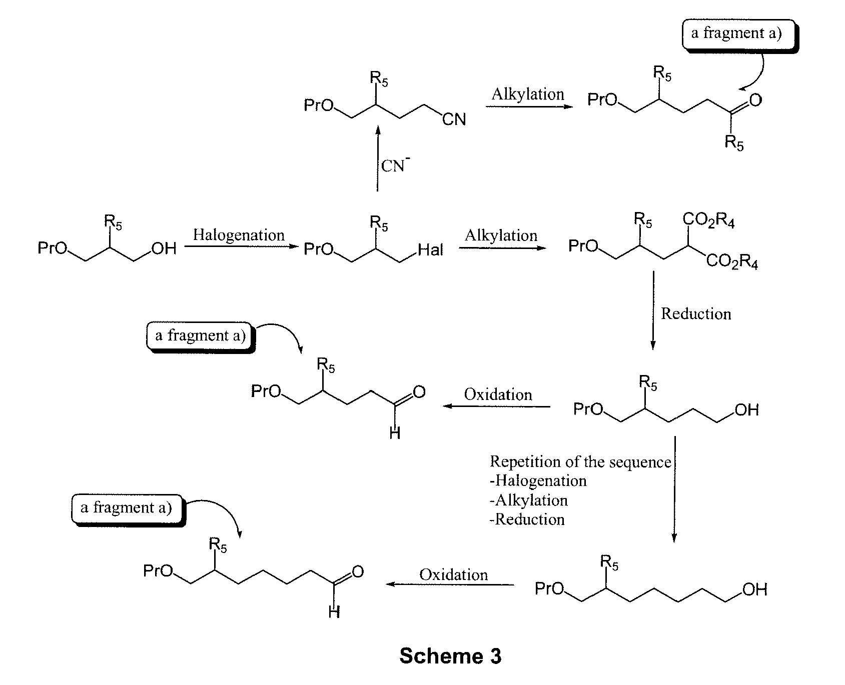

Fragment b) may be obtained by a Witting type reaction over 3-furancarboxaldehyde or 2-furancarboxaldehyde. Further chain elongations may performed analogous to those described above in schemes 1 and 2 (scheme 4):

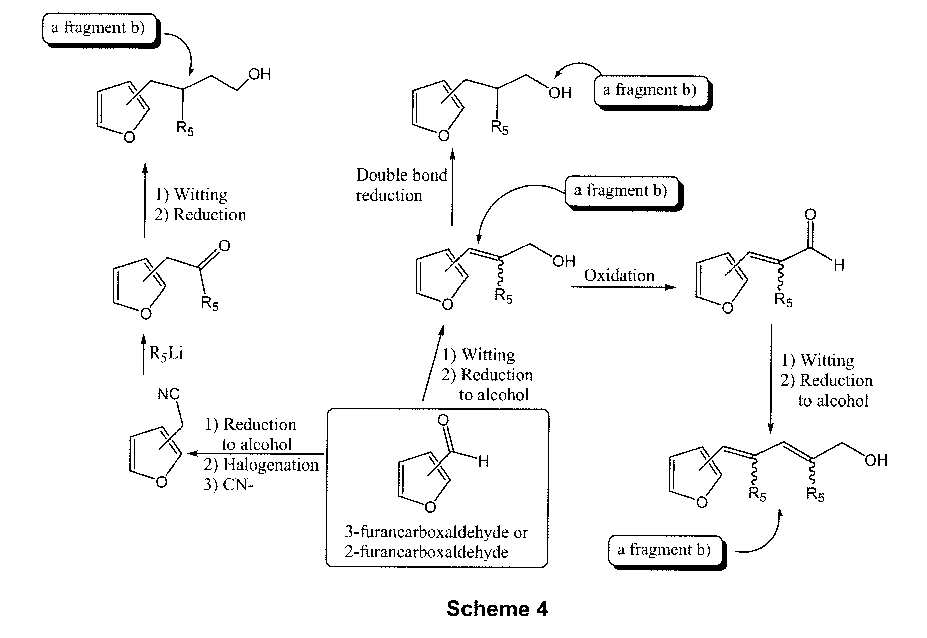

Prior to the coupling of fragments a) and b), fragment b) is transformed into the corresponding phosphorous ylide or sulphone, preferable a sulphone: 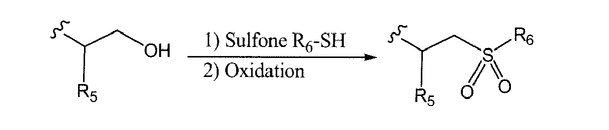 wherein R₆ is selected from the group consisting of substituted or unsubstituted alkyl, substituted or unsubstituted alkenyl, substituted or unsubstituted aryl, substituted or unsubstituted aralkyl, substituted or unsubstituted cycloalkyl, and substituted or unsubstituted heterocyclyl

Coupling is performed following common methods, such as dissolving the above mentioned sulphone in the presence of a strong base in order to obtain the corresponding nucleophilic carbon and further adding fragment a).

Thus, a further aspect of the present invention is a process for the synthesis of a compound of formula (I), its salts or solvates thereof, comprising in any order one or more of the steps selected from the group consisting of:
a) alkylation of furan with a compound of formula (XI) 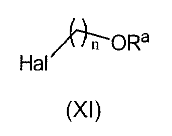 wherein R^{a} and n are as defined above and Hal is a halogen atom;
b) Witting type reaction comprising a compound selected from the group consisting of
   (i) 3-furancarboxaldehyde;
   (ii) 2-furancarboxaldehyde;
   (iii) a compound of formula (XII) 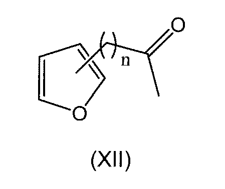 wherein n is as defined above; and
   (iv) a compound of formula (XIII) 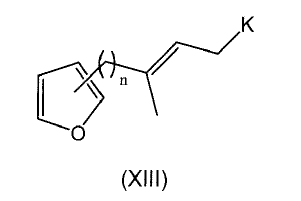
   wherein K is an aldehyde group or a sulphur ylide precursor; or
c) protection, alkylation or nucleophilic substitution on at least one hydroxyl group of a compound of a compound of formula (XV)

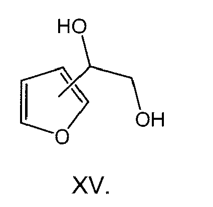

### Definitions

In the definition of the above mentioned compounds, the following terms have the meaning indicated:
Unless otherwise indicated, **"alkyl"** refers to a straight or branched hydrocarbon chain radical consisting of carbon and hydrogen atoms, containing no unsaturation, having one to twenty carbon atoms, preferably one to twelve, more preferably one to six, and which is attached to the rest of the molecule by a single bond, e. g., methyl, ethyl, n-propyl, i-propyl, n-butyl, t-butyl, n-pentyl, etc.
Unless otherwise indicated, "**alkenyl**" refers to a straight or branched hydrocarbon chain radical consisting of carbon and hydrogen atoms, containing at least one unsaturation, having two to twenty carbon atoms, preferably two to twelve carbon atoms, and which is attached to the rest of the molecule by a single bond, e. g., n-propenyl, i-propenyl, n-buten-2-yl, n-buten-3-yl, n-pent-2-yl, n-pent-3-yl, n-pent-4-yl, prenyl, poliprenyl, etc.
"**Alkynyl**" refers to a straight or branched hydrocarbon chain radical consisting of carbon and hydrogen atoms, containing at least one carbon-carbon triple bond, conjugated of not, having two to twenty carbon atoms, preferably two to twelve carbon atoms, and which is attached to the rest of the molecule by a single bond, such as - C≡CH, -CH₂C≡CH, -C≡CCH₃, -CH₂C≡CCH₃.
"**Aryl**" or "**Ar**" refers to a phenyl (also abbreviated as "Ph"), naphthyl, indenyl, fenanthryl or anthracyl radical, preferably phenyl or naphthyl radical.
"Aralkyl" refers to an aryl group linked to an alkyl group. Preferred examples include benzyl and phenethyl.
"**Cycloalkyl**" refers to a stable 3-to 10-membered monocyclic or bicyclic radical which is saturated or partially saturated, and which consist solely of carbon and hydrogen atoms. Suitable cycloalkyl groups include, but are not limited to cycloalkyl groups such as cyclopropyl. cyclobutyl, cyclobunyl, cyclopentyl or cyclohexyl.
**"Heterocyclyl"** refers to a stable 3- to 15-membered ring which consists of carbon atoms and from one to five heteroatoms selected from the group consisting of nitrogen, oxygen, and sulphur, preferably a 4- to 8-membered ring with one or more heteroatoms, more preferably a 5- or 6-membered ring with one or more heteroatoms. For the purposes of this invention, an heterocyclyl group may be a monocyclic, bicyclic or tricyclic ring system, which may include fused ring systems; and the nitrogen, carbon or sulfur atoms in the heterocyclyl radical may be optionally oxidised; the nitrogen atom may be optionally quaternized; and the heterocyclyl radical may be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole, tetrahydrofuran.

References herein to substituted groups in the compounds of the present invention refer to the specified moiety that may be substituted at one or more available positions by one or more suitable groups, e. g., an alkyl as defined above, halogen such as fluoro, chloro, bromo and iodo; cyano; hydroxyl; nitro; azido; -C(=O)OH;-C(=O)OR"; -C(=O)NH₂; -C(=O)NHR"; -C(=O)NR"R"'; -C(=O)R"; alkenyl and alkynyl groups including groups having one or more unsaturated linkages and from 2 to about 12 carbon or from 2 to about 6 carbon atoms; -OR"; -OAr, such as phenoxy; -SR"; alkylsulfinyl (alkyl-S(=O)-) groups including those moieties having one or more sulfinyl linkages and from 1 to about 12 carbon atoms or from 1 to about 6 carbon atoms; alkylsulfonyl groups including those moieties having one or more sulfonyl linkages and from 1 to about 12 carbon atoms or from 1 to about 6 carbon atoms; -NH₂; -NHR"; - NR"R'" or aryl; wherein R" and R'" are independently selected form alkyl or alkenyl radical as defined above. Unless otherwise indicated, an optionally substituted group may have a substituent at each substitutable position of the group, and each substitution is independent of the other.

"**Protecting group**" refers either hydroxyl protecting groups, amino protecting groups or thiol protecting groups as defined below.

"**Amino protecting group"** refers to a group that blocks the NH₂ function for further reactions and can be removed under controlled conditions. The amino protecting groups are well known in the art, representative protecting groups are
- amides of formula -C(=O)R', such as acetate amide, benzoate amide; pivalate amide; methoxyacetate amide; chloroacetate amide; levulinate amide;
- carbamates of formula -C(=O)-O-R', such as benzyl carbamate, p-nitrobenzyl carbamate, tert-butyl carbamate, 2,2,2-trichloroethyl carbamate, 2-(trimethylsilyl)ethyl carbamate, allyl carbamate; or.

In all the above formula R' represents a group selected from the group consisting of substituted of unsubstituted alkyl, substituted of unsubstituted alkenyl, substituted of unsubstituted alkynyl, substituted of unsubstituted aryl and substituted of unsubstituted aralkyl.

Also different alkyl moeties may be used as amino protecting groups.

Additional examples of amino protecting groups can be found in reference books such as Greene and Wuts' "Protective Groups in Organic Synthesis", John Wiley & Sons, Inc., New York, 4th Ed., 2007.

**"Thiol protecting group"** refers to a group that blocks the -SH function for further reactions and can be removed under controlled conditions. The same groups which are used as protecting groups of the hydroxyl moiety may be used as protecting groups of the -SH function. Preferred protecting groups are:
- silyl derivatives of formula -Si(R')₃, such as trimethylsilyl (also represented as "TMS"), triethylsilyl, tert-butyldimethylsilyl (also represented as "TBDMSO"), tert-butyldiphenylsilyl, tri-isopropylsilyl, diethylisopropylsilyl, thexyldimethylsilyl ether, triphenylsilyl, di-tert-butylmethylsilyl;
- thioethers of formula -R', such as methyl thioether, tert-butyl thioether, benzyl thioether, p-methoxybenzyl thioether, 3,4-dimethoxybenzyl thioether, trityl thioether; allyl thioether;
- thioesters of formula -C(=O)R', such as acetate thioester, benzoate thioester; pivalate thioester; methoxyacetate thioester; chloroacetate thioester; levulinate thioester;

In all the above formula R' represents a group selected from the group consisting of substituted of unsubstituted alkyl, substituted of unsubstituted alkenyl, substituted of unsubstituted alkynyl, substituted of unsubstituted aryl and substituted of unsubstituted aralkyl.

For further representative -SH protecting groups see pages 647-695 of Greene and Wuts' "Protective Groups in Organic Synthesis", John Wiley & Sons, Inc., New York, 4th Ed., 2007.

**"Hydroxyl protecting group"** refers to a group that blocks the -OH function for further reactions and can be removed *in vivo* or under controlled conditions. The hydroxyl protecting groups are well known in the art, representative protecting groups are:
- silyl derivatives of formula -Si(R')₃, such as trimethylsilyl (also represented as "TMS"), triethylsilyl, tert-butyldimethylsilyl (also represented as "TBDMSO"), tert-butyldiphenylsilyl, tri-isopropylsilyl, diethylisopropylsilyl, thexyldimethylsilyl, triphenylsilyl, di-tert-butylmethylsilyl;
- ethers of formula -R', such as methyl ether, tert-butyl ether, benzyl ether, p-methoxybenzyl ether, 3,4-dimethoxybenzyl ether, trityl ether; allyl ether;
- alkoxymethyl ethers of formula -CH₂-O-R', such as methoxymethyl ether, 2-methoxyethoxymethyl ether, benzyloxymethyl ether, p-methoxy-benzyloxymethyl ether, 2-(trimethylsilyl)ethoxymethyl ether. The oxygen atom may be replaced by a sulphur atom to form an alkylthiomethyl ether of formula -CH₂-S-R', such as methylthiomethyl ether. Tetrahydropyranyl and related ethers are also commonly used as hydroxyl protecting groups;
- esters of formula -C(=O)R', such as acetate ester, benzoate ester; pivalate ester; methoxyacetate ester; chloroacetate ester; levulinate ester;
- carbonates of formula -C(=O)-O-R', such as benzyl carbonate, p-nitrobenzyl carbonate, tert-butyl carbonate, 2,2,2-trichloroethyl carbonate, 2-(trimethylsilyl)ethyl carbonate, allyl carbonate; or
- sulphates such as SO₃-O-R' or its salts such as SO₃·py.

In all the above formula R' represents a group selected from the group consisting of substituted of unsubstituted alkyl, substituted of unsubstituted alkenyl, substituted of unsubstituted alkynyl, substituted of unsubstituted aryl and substituted of unsubstituted aralkyl.

Additional examples of hydroxyl protecting groups can be found in reference books such as Greene and Wuts' "Protective Groups in Organic Synthesis", John Wiley & Sons, Inc., New York, 4th Ed., 2007.

"**Halo**" refers to bromo, chloro, iodo or fluoro.

The compounds of the invention are also meant to include compounds which differ only in the presence of one or more **isotopically enriched atoms**. For example, compounds having the present structures except for the replacement of a hydrogen by a deuterium or tritium, or the replacement of a carbon by a ¹³C- or ¹⁴C-enriched carbon or ¹⁵N-enriched nitrogen are within the scope of this invention.

The compounds of the invention may be in crystalline form either as free compounds or as solvates (e.g. hydrates) and it is intended that both forms are within the scope of the present invention. Methods of solvation are generally known within the art. Suitable solvates are pharmaceutically acceptable solvates. In a particular embodiment the solvate is a hydrate.

The compounds of the present invention represented by the above described formula I may include enantiomers depending on the presence of chiral centres or isomers depending on the presence of multiple bonds (e.g. Z, E). The single isomers, enantiomers or diastereoisomers and mixtures thereof fall within the scope of the present invention.

In the following, the present invention is further illustrated by examples. They should in no case be interpreted as a limitation of the scope of the invention as defined in the claims.

### EXAMPLES

### EXAMPLE 1

### PREPARATION OF 2-[(2-TERT-BUTYLDIPHENYLSILYLOXY)ETHYL]FURAN

### 1.- Preparation of 2-(tert-butyldiphenylsilyloxy)ethanol.

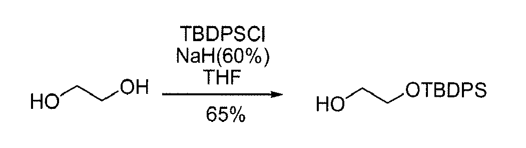

NaH (2.55 g; 13.15 mmol) was added to a solution of diol (3.96 g; 63.79 mmol) in THF (115 ml). The mixture was stirred for 45 minutes at room temperature, then TBDPSCI (3.96 g; 63.79 mmol) was added, left stirring under the same conditions for an additional 24 hours, after which time Et₂O (300 mL) was added, the organic phase was washed with a 10% K₂CO₃ aqueous solution (3 x 50 mL). The combined organic extracts were dried with Na₂SO₄, filtered and vacuum-concentrated. The raw product thus obtained was purified by column chromatography (mobile phase: 8% AcOEt/Hexane) obtaining monoprotected diol (12.43 g, 65%).

Yellow oil, Rf: 0.27 (20% AcOEt/Hexane).

**¹H-NMR (CDCl₃, δ):** 7.87 (4H, dd, J = 5.5 Hz, J = 2.2 Hz, CHₒ-Ph); 7.52 (6H, m, CH_{p,m}-Ph); 3.91 (2H, t, J = 5Hz, CH₂-1 ); 3.80 (2H, t, J = 5.1 Hz, CH₂-2); 2.11 (1H, s, OH); 1.27 (9H, s, CH₃-^{t}Bu).

**¹³C-NMR (CDCl₃, δ):** 135.28 (CHₒ-Ph); 133.19 (CHₚ-Ph); 129.51 (C-Ph); 127.52 (CHₘ-Ph); 64.37 (CH₂-2); 63.34 (CH₂-1); 26.67 (CH₃-^{t}Bu); 18.96 (C-^{t}Bu).

**MS (El⁺) [m/z, (%)]:** 301.08 ([M+1]⁺, 4); 243.05 ([M-^{t}Bu]⁺, 42); 224.10 (20); 223.10 (100); 199.07 (64); 197.08 (28); 165.11 (57).

**HRMS (EI⁺):** 301.1624 calculated for C₁₈H₂₅O₂Si and 301.1627 obtained.

### 2. Preparation of 1-(tert-butyldiphenylsilyloxy)-2-iodoethane.

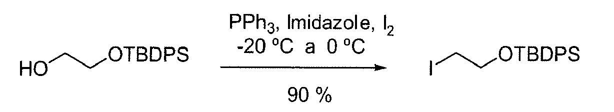

PPh₃ (4.20 g; 16.00 mmol) and imidazole (2.73 g; 39.90 mmol) were added to a solution of protected diol (3.99 g; 13.30 mmol) in THF (55 mL) and left stirring until dissolved; subsequently it was cooled to -20°C and then I₂ (3.86 g; 14.63 mmol) was added, an orange coloration being observed, stirring was maintained for 15 minutes, under the same conditions, then the cold bath was removed allowing the mixture to reach room temperature for 30 minutes, after which time and with the flask immersed in a water-ice bath, the reaction was stopped by adding a saturated NaHCO₃ solution (32 mL), the formation of a white precipitate being observed and the solution acquiring a transparent yellow color. The solid was separated by filtration, the precipitate was washed with Et₂O (50 mL) and the aqueous phase was extracted with Et₂O (3 x 50 mL). The pooled organic phases were washed with a 10% Na₂S₂O₄ solution (20 mL) and with H₂O (20 mL), they were dried with Na₂SO₄, filtered and vacuum-concentrated. The obtained raw product was purified by column chromatography (mobile phase: 100% CH₂Cl₂) obtaining iodide (4.88 g, 90%).

Yellow oil, Rf: 0.83 (100% CH₂Cl₂).

**¹H-NMR (CDCl₃,** δ**):** 7.75 (4H, m, CHₒ-Ph); 7.47 (6H, m, CH_{p,m}-Ph); 3.95 (2H, t, J = 6.8 Hz, CH₂-1); 3.29 (2H, t, J = 6.7 Hz, CH₂-2); 1;16 (9H, s, CH₃-^{t}Bu).

**¹³C-NMR (CDCl₃,** δ**):** 135.53 (CHₒ-Ph); 133.30 (C-Ph); 129.80 (CHₚ-Ph); 127.74 (CHₘ-Ph); 64.64 (CH₂-1); 26.80 (CH₃-^{t}Bu); 19.23 (C-^{t}Bu); 6.63 (CH₂-2).

**MS (FAB⁺) [m/z, (%)]:** 410.96 ([M+1]⁺, 7); 353.88 (21); 352.87 ([M-^{t}Bu]⁺, 100); 332.91 (23); 308.85 (96); 246.87 (27); 199.04 (31); 197.05 (40); 154.12 (36).

**HRMS (FAB⁺):** 411.0641 calculated for C₁₈H₂₄OSil and 411.0632 obtained.

### 3.- Preparation of 2-[(2-tert-butyldiphenylsilyloxy)ethyl]furan

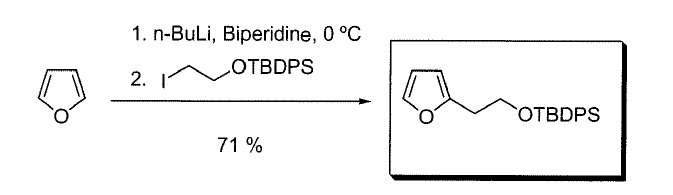

2.5 M n-BuLi in hexane (9.20 mL; 22.82 mmol) was slowly added to a solution of furan (1.59 g; 22.82 mmol) and 2,2'-bipyridine (catalytic quantity) in THF (40 mL), cooled to 0°C. The mixture was stirred for 1 hour under these conditions. After this time iodide (4.68 g; 11.41 mmol) dissolved in THF (7 mL) was added, and stirring was maintained at room temperature for 3 hours, after which time and with the flask immersed in a water-ice bath a saturated NH₄Cl solution (40 mL) was added. It was extracted with hexane (2 x 40 mL), the pooled organic extracts were dried with Na₂SO₄, filtered and vacuum-concentrated. The obtained raw product was purified by column chromatography (mobile phase: Hexane) obtaining alkyl furan (2.85 g, 71%).

Yellow oil, Rf: 0.70 (10% AcOEt/Hexane).

**¹H-NMR (CDCl₃,** δ**):** 7.85 (4H, m, CHₒ-Ph); 7.62 (6H, m, CH_{p,m}-Ph); 7.51 (1 H, s , CH-5 furyl); 6.25 (1H, s , CH-4 furyl); 5.96 (1H, s , CH-3 furyl); 4.12 (2H, t, J = 6.8 Hz, CH₂-1); 3.12 (2H, t, J = 6.7 Hz, CH₂-2); 1.26 (9H, s, CH₃-tBu).

**¹³C-NMR (CDCl₃,** δ**):** 153.69 (CH-2 furyl); 141.35 (CH-5 furyl); 135.98 (CHₒ-Ph); 134.17 (C-Ph); 130.01 (CHₚ-Ph); 128.06 (CHₘ-Ph); 110.62 (CH-4 furyl); 106.65 (CH-3 furyl); 62.79 (CH₂-2); 32.04 (CH₂-1); 27.20 (CH₃-^{t}Bu); 19.59 (C-^{t}Bu).

**MS (FAB⁺) [m/z, (%)]:** 351.08 ([M+1]⁺, 3); 293.04 ([M-^{t}Bu]⁺, 42); 273.08 (26).

**HRMS (FAB⁺):** 351.1780 calculated for C₂₂H₂₇O₂Si and 351.1762 obtained.

### EXAMPLE 2:

### PREPARATION OF (R)-2-(TERT-BUTYLDIPHENYLSILYLOXY)-1-(2-FURYL)-1-ETHANOL

### 1.- Preparation of (R)-2-(2-furyl)-1,2-ethanediol.

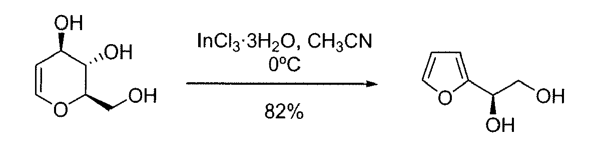

The experimental process was carried out following the description of the following publication;

Sobhana Babu B., Balsubramanian K.K., (2000). A Facile Synthesis of a Chiral Furan Diol from Glycals Catalyzed by Indium Trichloride. Journal of Organic Chemistry.; 65; 4198-4199.

### 2.- Preparation of (R)-2-(tert-butyldiphenylsilyloxy)-1-(2-furyl)-1-ethanol.

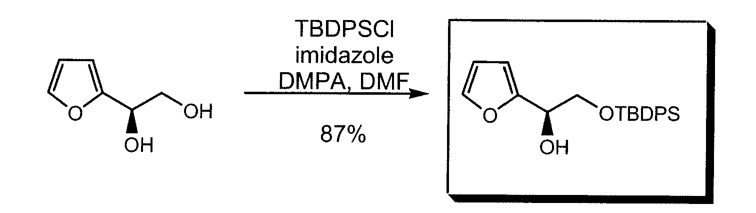

Imidazole (398 mg; 5.85 mmol), DMAP (catalytic) and TBDPSCI (1.52 mL; 5.85 mmol) were added to a solution of diol (680 mg; 5.32 mmol) in DMF (10 mL) and stirring was maintained for 23 h. After that time H₂O (10 mL) was added, it was extracted with Et₂O (3 x 20 mL) and washed with NaCl (sat.) (2 x 45 mL). The pooled organic extracts were dried with Na₂SO₄, filtered and vacuum-concentrated. A raw product was thus obtained which was purified by column chromatography (mobile phase: 10% AcOEt/Hexane), obtaining monoprotected diol (1.69 g, 87%).

Transparent oil, Rf: 0.70 (AcOEt).

**¹H-NMR (CDCl₃,** δ): 7.64 (4H, m, CHₒ-Ph); 7.40 (6H, m, CH_{p,m}-Ph); 7.35 (1 H, s, CH-5 furyl); 6.33 (1 H, s , CH-4 furyl); 6.30 (1 H, s , CH-3 furyl); 4.83 (1 H, d, J = 5.8 Hz, CH-1); 3.92 (2H, m, CH₂-2); 2.88 (1 H, s, -OH); 1.07 (9H, s, CH₃-tBu).

**¹³C-NMR (CDCl₃, δ):** 153.96 (CH-2 furyl); 142.45 (CH-5 furyl); 135.94 (CHₒ-Ph); 133.29 (C-Ph); 130.28 (CHₚ-Ph); 128.21 (CHₘ-Ph); 110.60 (CH-4 furyl); 107.50 (CH-3 furyl); 68.80 (CH-1); 66.79 (CH₂-2); 27.20 (CH₃-^{t}Bu); 19.64 (C-^{t}Bu).

**MS (EI⁺) [m/z, (%)]:** 309.09 ([M-^{t}Bu]⁺, 18); 200.06 (18); 199.05 (100).

**HRMS (EI⁺):** 309.0946 calculated for C₁₈H₁₇O₃Si and 309.0954 obtained.

### EXAMPLE 3:

### PREPARATION OF 1-TERT-BUTYLDIPHENYLSILYLOXY-6-(2-FURYL)HEXANE

### 1. Preparation of 1-(tert-butyldiphenylsilyloxy)-6-hexanol.

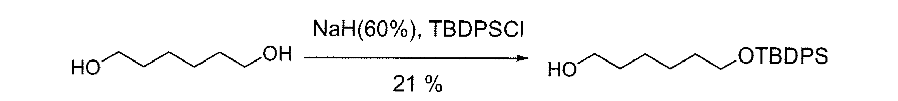

60% NaH (1.24 g) was dissolved in dry THF (56 mL) in a round-bottom flask provided with a magnetic bar and septum for argon inlet. A solution of diol (4.00 g, 33.84 mmoles) in dry THF was added to this mixture with a syringe. The mixture was stirred for 45 minutes at room temperature. After that time TBDPSCl (9.302 g, 33.84 mmoles) was added, maintaining stirring under the same conditions for an additional 120 hours. The preparation was carried out by adding Et₂O (300 mL) and a 10% K₂CO₃ aqueous solution (3x50 mL). The organic phase was dried with Na₂SO₄ and vacuum-concentrated. The obtained raw product was purified by pressure column chromatography (4 x 50 cm, mobile phase: 5% AcOEbHexane) obtaining monoprotected diol (2.58 g, 21 %).

Yellow oil, Rf: 0.30 (25% AcOEt/Hexane).

**¹H-NMR (CDCl₃,** δ**):** 7.95 (4H, m, CHₒ-Ph); 7.66 (6H, m, CH_{p,m}-Ph); 3.94 (2H, t, J= 6.5Hz, CH₂-1); 3.87 (2H, t, J= 6.5Hz, CH₂-6); 1.84 (5H, m, CH₂-2, CH₂-5, OH); 1.64 (4H, m, CH₂-3, CH₂-4); 1.33 (9H, s, CH₃-^{t}Bu).

**¹³C-NMR (CDCl₃, δ):** 135.52 (CHₒ-Ph); 134.07 (C-Ph); 129.53 (CHₚ-Ph); 127.53 (CHₘ-Ph); 63.80 (CH₂-1); 62.83 (CH₂-6); 32.67 (CH₂-2); 32.45 (CH₂-5); 26.83 (CH₃-^{t}Bu); 25.54 (CH₂-3); 25.42 (CH₂-4); 19.17 (C-^{t}Bu).

**MS (FAB⁺) [m/z, (%)]:** 357.15 ([M+1]⁺, 11); 299.08 ([M-^{t}Bu]⁺, 17); 224.10 (20); 199.07 (100); 197.08 (39).

**HRMS (FAB⁺):** 358.2328 calculated for C₂₂H₃₄O₂Si and 358.2317 obtained.

### 2. Preparation of 6-(tert-butyldiphenylsilyloxy)-1-iodohexane.

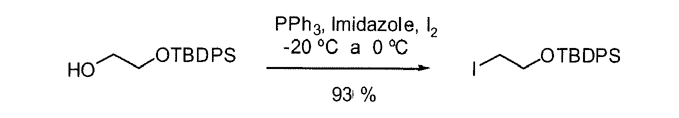

The monoprotected diol (1.90 g, 5.33 mmoles) was dissolved in dry THF (20 mL) in a round-bottom flask provided with a magnetic bar and septum for argon inlet. Then PPh₃ (1.76 g, 6.48 mmoles) and imidazole (1.14 g, 16.7 mmoles) were added and left stirring until dissolved. This solution was cooled to -20°C and then I₂ (1.56 g, 6.14 mmoles) was added, the solution turning an orange color. Stirring was stopped for 15 minutes at -20°C then it was left to reach room temperature for 30 minutes. The reaction was stopped with saturated NaHCO3 solution (20 mL), to that end the reaction flask is placed in a water-ice bath at 0°C, after the addition the solution turned from orange to transparent yellow, a white precipitate further appearing. The solution was vacuum-filtered to eliminate the white solid and the precipitate was washed with Et₂O (50 mL), the two phases which were formed were separated in a separating funnel and the aqueous phase was extracted with Et₂O (3 x 50 mL). Once the organic phases were pooled, they were washed with 10% solution of Na₂S₂O₄ (20 mL) and H₂O (20 mL). The organic phase was dried with Na₂SO₄ and vacuum-concentrated. The obtained raw product was purified by pressure column chromatography (4 x 24 cm, mobile phase: 100% CH₂Cl₂) obtaining iodide (2.30 g, 93%).

Yellow oil, Rf: 0.89 (100% CH₂Cl₂)].

**¹H-NMR (CDCl₃, δ):** 7.95 (4H, m, CHₒ-Ph); 7.61 (6H, m, CH_{p,m}-Ph); 3.81 (2H, t, J= 6.4Hz, CH₂-6); 3.23 (2H, t, J= 7Hz, CH₂-1); 1.89 (2H, t, J= 7Hz, CH₂-2); 1.69 (2H, t, J= 6.5Hz, CH₂-5); 1.49 (4H, m, CH₂-3, CH₂-4); 1.30 (9H, s, CH₃-^{t}Bu).

**¹³C-NMR (CDCl₃, δ):** 135.33 (CHₒ-Ph); 133.78 (C-Ph); 129.35 (CHₚ-Ph); 127.43 (CHₘ-Ph); 63.46 (CH₂-6); 33.27 (CH₂-2); 32.08 (CH₂-5); 29.98 (CH₂-3); 26.76 (CH₃-^{t}Bu); 24.53 (CH₂-4); 19.02 (C-^{t}Bu); 6.81 (CH₂-1).

**MS (FAB⁺) [m/z, (%)]:** 467.97 ([M+1]⁺, 4); 408.90 ([M-^{t}Bu]⁺, 20); 308.83 (66); 199.07 (100); 197.08 (73); 183.08 (44); 181.07 (28).

**HRMS (FAB⁺):** 467.1267 calculated for C₂₂H₃₂OSil and 467.1258 obtained.

### 3. Preparation of 1-tert-butyldiphenylsilyloxy-6-(2-furyl)hexane.

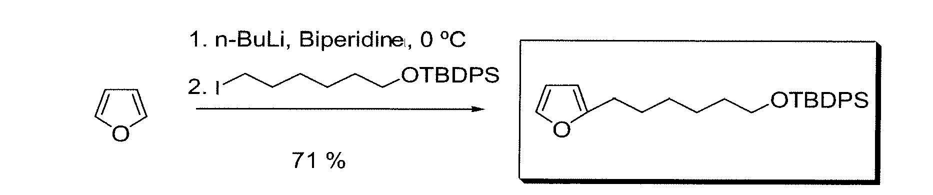

Furan (0.64 g, 9.38 mmoles) was dissolved in dry THF (38 mL) in a round-bottom flask provided with a magnetic bar and septum for argon inlet, and bipyridine (catalytic) was added. 2.5 M n-BuLi in hexane (3.75 mL, 9.38 mmoles) was slowly added to the solution previously cooled to 0°C. The reaction mixture was stirred for 1 hour at 0°C. After that time iodide (2.19 g, 4.69 mmoles) dissolved in dry THF (5 mL) was added. The mixture was stirred for 5 hours at room temperature after which time the reaction was stopped by adding a saturated NH₄Cl solution (20 mL) in a water-ice bath at 0°C, and hexane (20 mL) was added. The aqueous phase was separated from the organic, and the aqueous one is washed with hexane (20mL). The organic phases were extracted together with Na₂SO₄, filtered and vacuum-concentrated. The obtained raw product was purified by pressure column chromatography (4 x 20 cm, mobile phase: Hexane → 1% AcOEt/Hexane → 3% AcOEt/Hexane) obtaining alkyl furan (1.35 g, 70.5%).

Yellow oil, Rf: 0.71 (10% AcOEt/Hexane).

**¹H-NMR (CDCl₃,** δ**) :** 7.72 (4H, m, CHₒ-Ph); 7.43 (6H, m, CH_{p,m}-Ph); 7.33 (1 H, d, J= 1Hz , CH-5 furyl); 6.30 (1H, t , J= 2.45Hz, CH-4 furyl); 6.00 (1H, d, J= 3.0Hz, CH-3 furyl); 3.67 (2H, t, J = 6.4 Hz, CH₂-1); 2.63 (2H, t, J= 7.6Hz, CH₂-6); 1.60 (4H, m, CH₂-5, CH₂-2); 1.38 (4H, m, CH₂-4, CH₂-3); 1.09 (9H, s, CH₃-tBu).

**¹³C-NMR (CDCl₃,** δ**):** 156.49 (CH-2 furyl); 140.60 (CH-5 furyl); 135.54 (CHₒ-Ph); 134.06 (C-Ph); 129.47 (CHₚ-Ph); 127.55 (CHₘ-Ph); 110.00 (CH-4 furyl); 104.51 (CH-3 furyl); 63.83 (CH₂-1); 32.41 (CH₂-2); 28.87 (CH₂-6); 27.97 (CH₂-5); 27.88 (CH₂-4); 26.83 (CH₃-^{t}Bu); 25.48 (CH₂-3); 19.19 (C-^{t}Bu).

**MS (FAB⁺) [m/z, (%)]:** 407.17 ([M+1]⁺, 4); 349.08 ([M-^{t}Bu]⁺, 45). **HRMS (FAB⁺):** 407.2406 calculated for C₂₆H₃₅O₂Si and 407.2401 obtained.

### EXAMPLE 4:

### PREPARATION OF (E)-3-(3-FURYL)PROP-2-EN-1-OL

### 1. Preparation of ethyl (E)-3-(3-furyl)prop-2-enoate

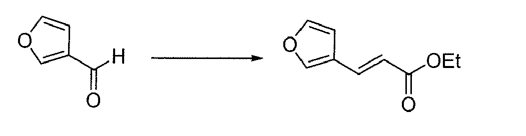

(Ethoxycarbonylmethylene)triphenylphosphorane (40.2 g; 115.3 mmol) was added to a solution of aldehyde (10.1 g; 104.7 mmol) in THF (280 mL) and the mixture was heated under reflux for 2 h. After that time and once the reaction reached room temperature, the solvent was vacuum-dried. The obtained residue was purified by column chromatography (mobile phase: 30% AcOEt/Hexane), providing the ester (17.3 g, 99%).

White solid, Pf: 38-40°C, Rf: 0.64 (50% AcOEt/Hexane).

**¹H-NMR (CDCl₃, δ):** 7.58 (1H, s, CH-5 furyl); 7.50 (1H, d, *J*= 5.8 Hz, CH-3); 7.36 (1 H, s, CH-2 furyl); 6.52 (1H, s, CH-4 furyl); 6.10 (1H, d, *J*= 5.8 Hz, CH-2); 4.18 (2H, c, *J*= 7.1 Hz, CH₂-OEt); 1.25 (3H, t, *J*= 7.1 Hz, CH₃-OEt).

**¹³C-NMR (CDCl₃, δ):** 166.78 (C=O); 144.32 (CH-3); 114.25 (CH-5 furyl); 134.38 (CH-2 furyl); 122.53 (C-3 furyl); 117.91 (CH-2); 107.31 (CH-4 furyl); 60.19 (CH₂-OEt); 14.16 (CH₃-OEt).

**MS (EI⁺) [m/z, (%)]:** 166.06 ([M]⁺, 67); 121.03 ([M-OEt]⁺, 100); 93.03 (30); 64.99 (28).

**HRMS (EI⁺):** 166.0630 calculated for C₉H₁₀O₃ and 166.0632 obtained.

### 2. Preparation of (E)-3-(3-furyl)prop-2-en-1-ol

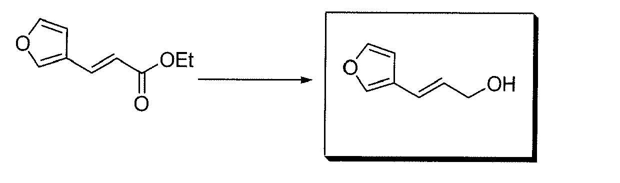

LiAlH₄ (11.9 g; 313 mmol) was added in small portions to a solution of the ester (17.3 g; 104 mmol) in Et₂O (61 mL), with the flask immersed in an ice bath and provided with condenser, observing that the mixture was under reflux. After the addition the cold bath was removed and stirring was maintained at room temperature for 1h. After that time with the flask immersed in an ice bath, H₂O was added until the end of the bubbling was observed, in this moment the mixture was stirred vigorously for 30 min. Then Na₂SO₄ was added, the aluminium salts were filtered and finally the filtered liquids were concentrated under reduced pressure, obtaining virtually pure allyl alcohol (12.3 g, 95%).

Colorless yellow liquid, Rf: 0.31 (50% AcOEt/Hexane).

**¹H-NMR (CDCl₃, δ):** 7.40 (1 H, s, CH-5 furyl); 7.35 (1 H, s, CH-2 furyl); 6.51 (1 H, s, CH-4 furyl); 6.46 (1H, d, *J*= 15.9 Hz, CH-3); 6.09 (1H, td, *J*= 13.8 Hz, *J*= 5.9 Hz, CH-2); 4.25 (2H, d, *J*= 5.9 Hz, CH₂-1).

**¹³C-NMR (CDCl₃,** δ**):** 143.58 (CH-5 furyl); 140.53 (CH-2 furyl); 128.11 (CH-2); 123.61 (C-3 furyl); 121.16 (CH-3); 107.55 (CH-4 furyl); 63.64 (CH₂-1).

**MS (EI⁺) [m/z, (%)]:** 124.13 ([M]⁺, 4); 123.12 ([M-1]⁺.9); 97.10 (22); 83.07 (26); 81.06 (36); 71.05 (32); 69.03 (68); 68.96 ([M-C₃H₃O]⁺, 100).

**HRMS (EI⁺):** 123.0810 calculated for C₇H₈O₂ and 123.0802 obtained.

### EXAMPLE 5:

### PREPARATION OF METHYL (E)-6-(3-FURYL)-3-METHYLHEX-2-ENOATE

### 1. Preparation of 3-(3-furyl)propan-1-ol.

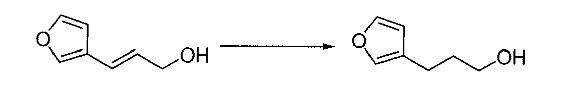

A suspension of (*E*)-3-(3-furyl)prop-2-en-1-ol (4.1 g; 33 mmol), obtained from **EXAMPLE 4**, and Pd/C (10%) (0.32 g; 0.3 mmol) in MeOH (30 mL), was vigorously stirred under H₂ atmosphere for 7 h. After that time in order to remove the catalyst it was filtered over celite and the filtered liquids were concentrated with reduced pressure, obtaining alcohol (4.1 g, 98%).

Colorless liquid, Rf: 0.31 (50% AcOEt/Hexane).

**¹H-NMR (CDCl₃,** δ**):** 7.34 (1 H, s, CH-5 furyl); 7.23 (1H, s, CH-2 furyl); 6.27 (1 H, s, CH-4 furyl); 3.68 (2H, t, *J=* 6.27 Hz, CH₂-1); 2.51 (2H, t, *J=* 7.66 Hz, CH₂-3); 1.82 (2H, q, *J=* 7.10 Hz, CH₂-2); 1.57 (1H, b.s., -OH).

**¹³C-NMR (CDCl₃,** δ**):** 142.85 (CH-5 furyl); 138.89 (CH-2 furyl); 124.39 (C-3 furyl); 110.92 (CH-4 furyl); 62.27 (CH₂-1); 32.81 (CH₂-2); 21.01 (CH₂-3).

**MS (EI⁺) [m/z, (%)]:**126.07 ([M]⁺, 20); 125.06 ((M-1]⁺.100); 113.06 (36); 112.05 (20); 111.05 (21); 109.7 ([M-OH]⁺, 15); 97.06 (20); 95.05 ([M-CH₃O]⁺, 20); 82.03 (28); 81.02 ([M-C₂H₅O]⁺, 39); 79.04 (25); 67.02 ([M-C₃H₇O]⁺, 31).

**HRMS (EI⁺):** 125.0603 calculated for C₇H₉O₂ and 125.0607 obtained.

### 2. Preparation of 3-(3-furyl)-1-iodopropane.

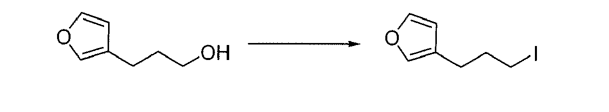

PPh₃ (2.16 g; 8.25 mmol) and imidazole (1.4 g; 20.6 mmol) were added to a solution of alcohol (0.87 g; 6.9 mmol) in THF (50 mL) and were left stirring at room temperature until completely dissolved, subsequently it was cooled to -20°C and iodine (2.26 g ;8.9 mmol) was added, the appearance of an orange color being observed, the stirring was maintained for 15 min under the same conditions and then the cold bath was removed allowing the mixture to reach room temperature for 30 min, after that time and with the flask immersed in an ice bath the reaction was stopped by adding a saturated NaHCO3 solution (49 mL), then the formation of a white precipitate was observed and the solution acquired a transparent yellow color. The solid was separated by filtration and the aqueous phase was extracted with TBME (3 x 50 mL). The pooled organic phases were washed with a 10% Na₂SO₃ solution (150 mL) and H₂O (150 mL), they were dried, filtered and vacuum-concentrated. The raw product thus obtained was purified by column chromatography (mobile phase: CH₂Cl₂), obtaining iodide (1.3 g, 80%).

Yellow liquid, Rf: 0.80 (50% AcOEt/Hexane).

**¹H-NMR (CDCl₃, δ):** 7.35 (1H, t, *J*= 1.5 Hz, CH-5 furyl); 7.25 (1H, s, CH-2 furyl); 6.26 (1H, s, CH-4 furyl); 3.18 (2H, t, *J*= 6.68 Hz, CH₂-1); 2.54 (2H, t, *J*= 7.24 Hz, CH₂-3); 1.08 (2H, q, *J*= 6.96 Hz, CH₂-2)

**¹³C-NMR (CDCl₃, δ):** 143.00 (CH-5 furyl); 139.28 (CH-2 furyl); 122.97 (C-3 furyl); 110.76 (CH-4 furyl); 33.35 (CH₂-2); 25.33 (CH₂-3); 6.28 (CH₂-1).

**MS (FAB⁺) [m/z, (%)]:** 235.98 ([M]⁺, 36); 221.11 (37); 219.17 (26); 217.16 (21); 207.11 (34); 205.17 (28); 203.17 (27); 202.17 (20); 193.17 (29); 191.18 (40); 181.18 (28); 177.22 (36); 169.12 (35); 167.20 (40); 165.22 (65); 163.25 (54); 161.24 (47); 159.23 (52); 155.22(64); 154.20 (100); 151.33 (43); 151.17 (50); 151.07 (54).

**HRMS (FAB⁺):** 235.9698 calculated for C₇H₉Ol and 235.9759 obtained.

### 3. Preparation of 4-(3-furyl)-1-butanenitrile.

### 3.1 Method A:

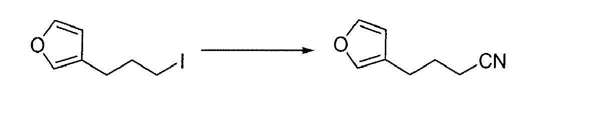

NaCN (324 mg; 6.5 mmol) was added to a solution of iodide (1.1 g; 4.6 mmol) in DMSO (12 mL) and the resulting mixture was heated at 90°C for 1 h. After that time and once room temperature was reached, H₂O (50 mL) was added and it was extracted with TBME (3 x 50 mL). The pooled organic extracts were dried, filtered and vacuum-concentrated, obtaining virtually pure nitrile (625 mg, 99%).

Colorless liquid, Rf: 0.70 (50% AcOEt/Hexane).

**¹H-NMR (CDCl₃,** δ**):** 7.37 (1H, t, *J=* 1.75 Hz, CH-5 furyl); 7.26 (1H, dd, *J=* 1.59 Hz, *J=* 0.95 Hz, CH-4 furyl); 6.25 (1H, s, CH-2 furyl); 2.60 (2H, t, *J=* 7.30 Hz, CH₂-2); 2.33 (2H, t, *J=* 7.15 Hz, CH₂-4); 1.91 (2H, q, *J=* 7.31 Hz, CH₂-3)

**¹³C-NMR (CDCl₃,** δ**):** 134.31 (CH-5 furyl); 139.41 (CH-2 furyl); 122.53 (C-3 furyl); 119.36 (C-1); 110.49 (CH-4 furyl); 25.65 (CH₂-3); 23.46 (CH₂-4); 16.35 (CH₂-2)

**MS (EI⁺) [m/z, (%)]:**135.07 ([M]⁺, 20); 95.05 ([M-C₂H₂N]⁺, 5); 82.04 (30); 81.03 ([m-C₃H₄N]⁺, 100).

**HRMS (EI⁺):** 135.0684 calculated for C₈H₉NO and 135.0685 obtained.

### 3.2 Method B.

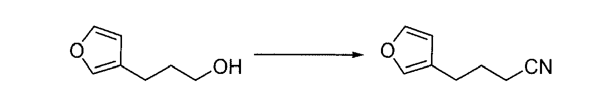

A 2.5 M solution of n-BuLi in Hexane (14.3 mL; 35.8 mmol) was added dropwise to a solution of the alcohol (3.8 g; 29.8 mmol) in THF (150 mL) cooled to 0°C. After finishing the addition stirring was extended for 10 min. After that time p-TsCl (8.5 g; 44.7 mmol) was added and stirring was maintained for another 2 h under the same conditions. Then H₂O (100 mL) was added and it was extracted with AcOEt (3 x 150 mL). The pooled organic phases were dried, filtered and vacuum-concentrated. The residue thus obtained was dissolved in DMSO (80 mL) and NaCN (2.3 g; 45.5 mmol) was added. The resulting mixture was heated to 90°C for 2h, after which time and once room temperature was reached, H₂O (100 mL) was added and it was extracted with TBME (3 x 150 mL). The pooled organic extracts were dried, filtered and vacuum-concentrated. The raw product thus obtained was purified by column chromatography (mobile phase: 3% AcOEt/Hexane), providing the nitrile (3.35 g, 85%).

### 4. Preparation of 5-(3-furyl)-2-pentanone.

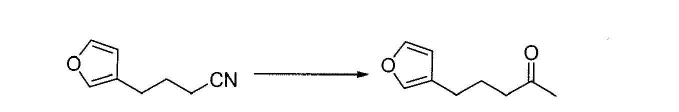

A 1.5 M MeLi solution in Et₂O (4.8 mL; 7.23 mmol) was added to a solution of nitrile (326.5 mg; 2.4 mmol) in Et₂O (5.7 mL) cooled to 0°C, and after finishing the addition, the mixture was stirred for 30 min. After that time a saturated NH₄Cl solution was added, until the bubbling stopped, then it was extracted with AcOEt (3 x 20 mL). The pooled organic phases were dried, filtered and vacuum-concentrated. The obtained residue was purified by column chromatography (mobile phase: 3% AcOEt/Hexane), and yielded the ketone (332 mg, 85%).

Colorless liquid, Rf: 0.65 (50% AcOEt/Hexane).

**¹H-NMR (CDCl₃, δ):** 7.32 (1H, s, CH-5 furyl); 7.18 (1H, s, CH-2 furyl); 6.23 (1H, s, CH-4 furyl); 2.40 (2H, c, *J=* 7.10 Hz, CH₂-3, CH₂-5); 2.09 (3H, s, CH₃-1); 1.80 (2H, q, *J=* 7.4 Hz, CH₂-4).

**¹³C-NMR (CDCl₃, δ):** 208.61 (C=O); 142.80 (CH-5 furyl); 138.93 (CH-2 furyl); 124.17 (C-3 furyl); 110.78 (CH-4 furyl); 42.76 (CH₂-5); 29.87 (CH₃-1); 23.92 and 23.79 (CH₂-3, CH₂-4)

**MS (El⁺) [m/z, (%)]:** 152.08 ([M]⁺, 6); 134.07 ([M-H₂O]⁺.12); 94.04 ([M-C₃H₆O]⁺, 100)

**HRMS (EI⁺):** 152.0837 calculated for C₉H₁₂O₂ and 152.0832 obtained.

### 5. Preparation of methyl (E)-6-(3-furyl)-3-methylhex-2-enoate.

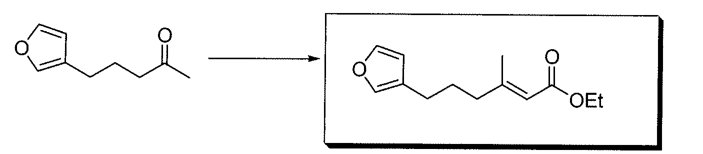

(Ethoxycarbonylmethylene)diethylphosphonate (16.5 mL; 82.9 mmol) was added to a 60% NaH solution (2.8 g; 70.8 mmol) in THF (70 mL) cooled to 0°C. After finishing the addition the cold bath was removed and the mixture was stirred for 18 h at room temperature. After that time and at the same temperature the ketone (1.8 g; 11.8 mmol) dissolved in THF (10 mL) was added, the stirring was maintained under the same conditions for 24 h, after which time the reaction was stopped by adding saturated NH₄Cl solution and it was extracted with AcOEt (3 x 100 mL). The pooled organic extracts were dried, filtered and vacuum-concentrated. The raw product thus obtained was purified by column chromatography (mobile phase: 10% AcOEt/Hexane), yielding the ester (2.6 g, 99%).

Colorless liquid, Rf: 0.73 (100% AcOEt).

**¹H-NMR (CDCl₃,** δ**):** 7.34 (1H, s, CH-5 furyl); 7.20 (1H, s, CH-2 furyl); 6.24 (1 H, s, CH-4 furyl); 5.65 (1H, s, CH-2); 4.13 (2H, c, *J=* 6.9 Hz,CH₂-OEt); 2.44 (2H, td, *J=* 20.0 Hz, *J=* 7.62 Hz, CH₂-6); 2.14 (5H, m, CH₃-3, CH₂-4); 1.72 (2H, q, *J=* 7.5 Hz, CH₂-5); 1.26 (3H, t, *J=* 7.2 Hz, CH₃-OEt)

**¹³C-NMR (CDCl₃,** δ**):** 166.82 (C=O); 159.46 (C-3); 142.84 (CH-5 furyl); 138.92 (CH-2 furyl); 124.43 (C-3 furyl); 115.85 (CH-2); 110.81 (CH-4 furyl); 59.50 (CH₂-OEt); 40.28 (CH₂-4); 27.61 (CH₂-5); 24.20 (CH₂-6); 18.71 (CH₃-3); 14.31 (CH₃-OEt)

**MS (FAB⁺) [m/z, (%)]:** 223.16 ([M+1]⁺.44); 222.14 ([M]⁺.25); 221.15 (39); 219.18 (22); 217.16 (22); 215.13 (24); 209.14 (30); 207.15 (34); 203.17 (25); 202.16 (26); 193.16 (100); 192.15 (34); 191.17 (50); 177.21 (63); 163.24 (67); 155.23 (43); 154.21 (66); 151.36 (51); 151.16 (59).

**HRMS (FAB⁺):** 223.1334 calculated for C₁₃H₁₉O₃ and 223.134 obtained.

### EXAMPLE 6:

### PREPARATION OF (8S)-(4E,6Z)-11-(tert-butyldiphenylsilyloxy)-1-(3-furyl)-4,8-dimethylundecan-4,6-diene

### 1. Preparation of (E)-6-(3-furyl)-3-methylhex-2-en-1-ol .

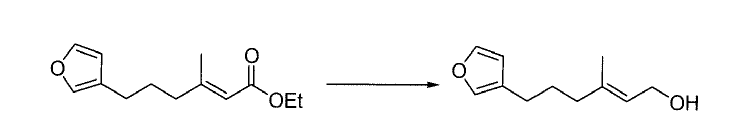

A 1.0 M Dibal-H solution in hexane (7.7 mL; 7.7 mmol) was added to the methyl (*E*)-6-(3-furyl)-3-methylhex-2-enoate (684 mg; 3.1 mmol) solution, obtained in **EXAMPLE 5**, in CH₂Cl₂ (20 mL) at a temperature of -78°C, and stirring was maintained for 30 min. After that time TBME (4 mL) and H₂O (0.5 mL) were added, the cold bath was removed and it was maintained with vigorous stirring until the formation of a white gel, in this moment H₂O (0.5 mL) and a 4.0 M solution NaOH (0.5 mL) were added, and stirring was maintained until the formation of a white precipitate, Na₂SO₄ was added and the salts were separated by vacuum filtration, obtaining virtually pure allyl alcohol (589 mg, 99%).

Colorless liquid, Rf: 0.65 (50% AcOEt/Hexane).

**¹H-NMR (CDCl₃, δ):** 7.33 (1H, s, CH-5 furyl); 7.20 (1 H, s, CH-2 furyl); 6.25 (1 H, s, CH-4 furyl); 5.41 (1H, ct, *J*= 1.25 Hz, *J*= 6.9 Hz, CH-2); 4.15 (2H, d, *J*= 6.9 Hz, CH₂-1); 2.39 (2H, t, *J*= 7.6 Hz, CH₂-6); 2.05 (2H, t, *J*= 7.7 Hz, CH₂-4); 1.69 (2H, t, *J*= 7.5 Hz, CH₂-5); 1.66 (3H, s, CH₃-3).

**¹³C-NMR (CDCl₃, δ):** 142.78 (CH-5 furyl); 139.46 (C-3); 138.82 (CH-2 furyl); 124.88 (C-3 furyl); 123.63 (CH-2); 110.90 (CH-4 furyl); 59.38 (CH₂-1); 39.00 (CH₂-4); 27.90 (CH₂-5); 24.31 (CH₂-6); 16.17 (CH₃).

**MS (EI⁺) [m/z, (%)]:** 180.11 ([M]⁺, 4); 95.03 (30); 94.02 ([M-C₅H₁₀O]⁺, 91); 82.00 ([M-C₆H₁₂O]⁺, 100); 71.00 (24).

**HRMS (EI⁺):** 180.1150 calculated for C₁₁H₁₆O₂ and 180.1145 obtained.

### 2. Preparation of benzo[d]thiazolyl and (E)-6-(3-furyl)-3-methyl hex-2-enyl sulfide.

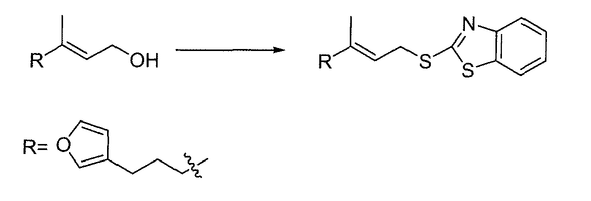

2-mercaptobenzothiazole (43 mg; 0.26 mmol) and PPh₃ (90 mg; 0.27 mmol) were added to a solution of the allyl alcohol (44 mg; 0.24 mmol) in THF (1 mL), then an ice bath was placed and 95% DIAD (54.6 µL; 0.26 mmol) was added. After finishing the addition stirring was extended under these conditions for 30 min. After that time and after vacuum evaporating the solvent a raw product was obtained which was purified by column chromatography (mobile phase: 1% AcOEt/Hexane), yielding the sulfide (72.1 mg, 91%).

Colorless liquid, Rf: 0.55 (30% AcOEt/Hexane).

**¹H-NMR (CDCl₃,** δ**):** 7.87 (1H, d, *J=* 8 Hz, CH-5'); 7.74 (1H, d, *J=* 8.2 Hz, CH-8'); 7.27 (4H, m, CH-6', CH-7', CH-5 furyl, CH-2 furyl); 6.22 (1H, m, CH-4 furyl); 5.49 (1H, m, CH-2); 4.01 (2H, d, *J*= 7.94 Hz, CH₂-1); 2.35 (4H, m, CH₂-4, CH₂-6); 1.76 (3H, s, CH₃-3); 1.69 (2H, c, *J*= 7.35 Hz, CH₂-5).

**¹³C-NMR (CDCl₃, δ):** 166.80 (C-2'); 153.34 (C-4'); 142.74 (CH-5 furyl); 141.59 (C-3); 138.82 (CH-2 furyl); 135.31 (C-9'); 125.96 (CH-8'); 124.73 (C-3 furyl); 124.12 (CH-5'); 121.45 (CH-7'); 120.90 (CH-6'); 118.05 (CH-2); 110.89 (CH-4 furyl); 38.90 (CH₂-4); 31.78 (CH₂-1); 27.76 (CH₂-5); 24.05 (CH₂-6); 16.21 (CH₃-3)

**MS (EI⁺) [m/z, (%)]::** 329.09 ([M]⁺, 3);167.99 (24); 166.07 ([M-C₇HNS₂]⁺, 100); 147.08 (33); 108.00 (34); 94.04 (25); 91.05 (20); 81.03 ([M-C₁₃H₁₄NS₂]⁺, 95); 79.05 (20).

**HRMS (EI⁺):** 329.0908 calculated for C₁₈H₁₉NOS₂ and 329.0914 obtained.

### 3. Preparation of benzo[d]thiazolyl-(E)-6-(3-furyl)-3-methylhex-2-enyl sulfone.

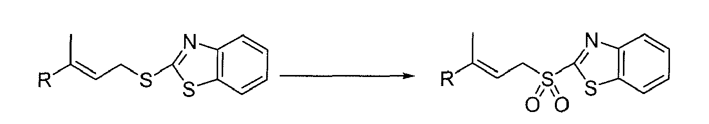

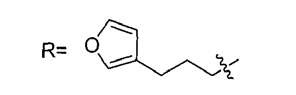

Mo₇O₂₄(NH₄)·4H₂O (262 mg ;45 µmol) and a 30% H₂O₂ solution (280 µL; 2.5 mmol) were added to a solution of sulfide (74 mg; 0.23 mmol) in EtOH (1.3 mL) cooled to 0°C, then the stirring was maintained for 6 h. After that time a saturated NH₄Cl solution was added and it was extracted with TBME (3 x 10 mL). The pooled organic extracts were dried, filtered and vacuum-concentrated, obtaining the sulfone (73 mg, 90%).

White solid, Pf: 58-60°C, Rf: 0.55 (30% AcOEt/Hexane)

**¹H-NMR (CDCl₃,** δ**):** 8.20 (1H, d, *J*= 7.94 Hz, CH-5'); 7.97 (1H, d, *J*= 8.63 Hz, CH-8'); 7.60 (2H, sex., *J*= 8.21 Hz, CH₂-6', CH₂-7'); 7.30 (1H, s, CH-5 furyl); 7.17 (1H, s, CH-2 furyl); 6.16 (1H, s, CH-4 furyl); 5.26 (1H, t, *J*= 7.8 Hz, CH-2); 4.24 (2H, d, *J*= 7.5 Hz, CH₂-1); 2.26 (2H, t, *J*= 7.6 Hz, CH₂-6); 2.03 (2H, t, *J*= 7.7 Hz, CH₂-4); 1.54 (5H, m, CH₃-3, CH₂-5).

**¹³C-NMR (CDCl₃, δ):** 165.89 (C-2'); 152.72 (C-4'); 148.03 (C-3); 142.69 (CH-5 furyl); 138.85 (CH-2 furyl); 136.95 (C-9'); 127.91 (CH-8'); 127.61 (CH-5'); 125.37 (CH-7'); 124.49 (C-3 furyl); 122.27 (CH-6'); 110.81 (CH-4 furyl); 108.89 (CH-2); 54.70 (CH₂-1); 39.06 (CH₂-4); 27.68 (CH₂-5); 23.94 (CH₂-6); 16.60 (CH₃-3).

**MS (EI⁺) [m/z, (%)]:** 163.11 ([M-C₇H₄NO₂S₂]⁺, 56); 147.08 (36); 135.01 (100); 108.00 (25); 94.04 (25); 81.04 ([M-C₁₃H₁₄NO₂S₂]⁺.92).

**HRMS (EI⁺):** 162.1045 calculated for C₁₁H₁₄O and 162.1052 obtained.

### 4. Preparation of methyl (R)-3-(tert-butyldimethylsilyloxy)-2-methylpropanoate

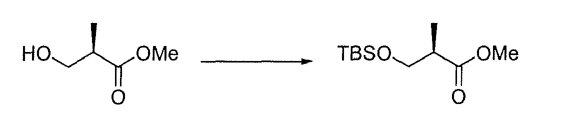

Imidazole (1.2 g; 16.9 mmol), DMAP (c.c.) and TBSCI (1.39 g; 9.24 mmol) were added successively to a solution of the alcohol (1 g; 8.4 mmol) in DMF (50 mL) and left stirring at room temperature for 4 h. After that time a saturated NH₄Cl solution (75 mL) was added and it was extracted with TBME (3 x 100 mL). The pooled organic extracts were dried, filtered and vacuum-concentrated. A residue was thus obtained which was purified by column chromatography (mobile phase: 10% AcOEt/Hexane), isolating the protected alcohol (1.9 g, 96%).

Colorless liquid, Rf: 0.73 (50% AcOEt/Hexane).

**¹H-NMR (CDCl₃, δ):** 3.76 (1H, dd, *J=* 9.8 Hz, *J=* 6.9 Hz, CH-3); 3.66 (3H, s, - OCH₃); 3.63 (1H, dd, *J=* 10.5 Hz, *J=* 6.1 Hz, CH-3); 2.63 (1H, c, *J=* 6.7 Hz, CH-2); 1.12 (3H, d, *J=* 7.1 Hz, CH₃-2); 0.85 (9H, s, CH₃-^{t}Bu); 0.02 (6H, s, CH₃-Si).

**¹³C-NMR (CDCl₃, δ):** 175.45 (C=O); 65.23 (CH₂-3); 51.48 (OCH₃); 42.53 (CH-2); 25.76 (CH₃-^{t}Bu); 18.19 (C-Si); 13.44 (CH₃-2); -5.52 ((CH₃)₂-Si).

**MS (FAB⁺) [m/z, (%)]:** 217.13 ([M-O]⁺, 12); 201.12 ([M-OMe]⁺, 44); 176.03 (65); 175.03 ([M-^{t}Bu]⁺, 97); 147.08 (62); 119.83 (35); 119.03 (96); 115.08 (20); 105.08 (32); 91.04 (20); 90.04 (36); 89.72 (68); 89.01 (100); 75.02 (73); 73.04 (78).

**HRMS (FAB⁺):** 217.1260 calculated for C₁₀H₂₁O₃Si and 217.1270 obtained.

### 5. Preparation of (R)-3-(tert-butyldimethylsilyloxy)-2-methylpropan-1-ol

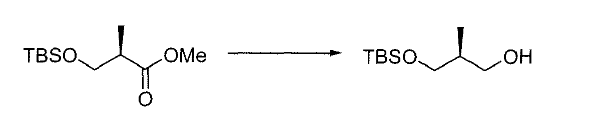

A 1.0 M Dibal-H solution in hexane (18.6 mL; 18.6 mmol) was added to a solution of ester (1.4 g; 6.2 mmol) in CH₂Cl₂ (35 mL) cooled to -78°C, and stirring was maintained for 30 min. After that time TBME (9 mL) and H₂O (1.3 mL) was added to the reaction mixture and left stirring vigorously allowing the mixture to room temperature until the formation of a white gel, in this moment H₂O (1.3 mL) and a 4.0 M NaOH solution (1.3 mL) were added, and the stirring was extended until the formation of a white precipitate, then Na₂SO₄ was added, the solids were separated by vacuum filtration, after the evaporation to dryness of the filtered liquids a residue was obtained which was purified by column chromatography (mobile phase: 40% AcOEt/Hexane), providing the alcohol (1.35 g, 99%).

Colorless liquid, Rf: 0.15 (10% AcOEt/Hexane).

**¹H-NMR (CDCl₃, δ):** 3.72-3.48 (4H, m,CH₂-1, CH₂-3); 2.80 (1H, b.s., -OH); 1.90 (1 H, m, CH-2); 0.87 (9H, s, CH₃-^{t}Bu); 0.76 (3H, d, *J*= 6.9 Hz, CH₃-2); 0.04 (6H, s, CH₃-Si).

**¹³C-NMR (CDCl₃, δ):** 68.53 (CH₂-3); 68.05 (CH₂-1); 37.05 (CH-2); 25.80 (CH₃-^{t}Bu); 18.12 (C-Si); 13.04 (CH₃-2); -5.06 (CH₃-Si).

**MS (FAB⁺) [m/z, (%)]:** 205.31 ([M+1]⁺, 33); 203.12 (56); 199.05 (38); 187.15 ([M-OH]⁺, 85); 189.13 ([M-CH₃]⁺, 18).

**HRMS (FAB⁺):** 205.1624 calculated for C₁₀H₂₅O₂Si and 205.1623 obtained.

### 6. Preparation of (R)-3-(tert-butyldimethylsilyloxy)-2-methyl-1-iodopropane.

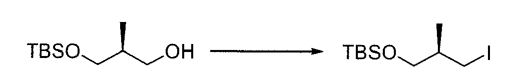

PPh₃ (1.3 g; 4.8 mmol) and imidazole (0.82 g; 12 mmol) were added to a solution of the alcohol (0.82 g; 4 mmol) in THF (50 mL) and stirred until completely dissolved, then cooled to -20°C and iodine (1.12 g; 4.4 mmol) was added, the appearance of an orange color being observed. The stirring was extended under the same conditions for 15 min, after that time the cold bath was removed and it was stirred for 1 h and 30 min, then with the flask immersed in an ice bath a saturated NaHCO₃ solution (24 mL) at 0°C was added, the formation of a white precipitate being observed and the solution acquired a transparent yellow color. The solid was separated by filtration and the aqueous phase was extracted with TBME (3 x 50 mL). The pooled organic phases were washed with a 10% Na₂SO₃ solution (100 mL) and with H₂O (100 mL), they were dried, filtered and vacuum-concentrated. The raw product thus obtained was purified by column chromatography (mobile phase: CH₂Cl₂), providing the iodide (1.1 g, 88%),

Yellow liquid, Rf: 0.85 (50% AcOEt/Hexane).

**¹H-NMR (CDCl₃, δ):** 3.51 (1H, c, *J*= 5.0 Hz, CH-3); 3.38 (1H, c, *J*= 6.9 Hz, CH-3); 3.26 (2H, dc, *J*= 23.8 Hz, *J*= 4.95 Hz, CH₂-1); 1.63 (1H, c, *J*= 6.26 Hz, CH-2); 0.93 (3H, d, *J*= 6.7 Hz, CH₃-2); 0.88 (9H, s, CH₃-^{t}Bu); 0.05 (6H, s, CH₃-Si).

**¹³C-NMR (CDCl₃, δ):** 66.70 (CH₂-3); 37.39 (CH-2); 25.89 ((CH₃)₃); 18.26 (C-Si); 17.24 (CH₃-2); 13.81 (CH₂-1); -5.39 ((CH₃)₂-Si).

**MS (FAB⁺) [m/z, (%)]:** 285.33 ([M-2CH₃]⁺, 17); 257.28 ([M-^{t}Bu]⁺, 21); 155.23 (30).

**HRMS (FAB⁺):** 256.9859 calculated for C₆H₁₄IOSi and 257.0241 obtained.

### 7. Preparation of dimethyl (R)-4-(tert-butyldimethylsilyloxy)-3-methylbutane-1,1-dicarboxilate.

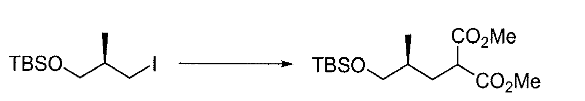

Dimethyl malonate (518 µL; 4.44 mmol) was added to a 60% NaH dispersion (178 mg; 4.44 mmol) in DMF (0.5 mL) and THF (3 mL), a white foam being observed, then a solution of the iodide (698 mg; 2.22 mmol) in THF (7 mL) was added and the mixture was heated under reflux for 18 h. After that time it was allowed to reach room temperature, AcOEt (50 mL) was added and the organic phase was washed with saturated NaCl solution (2 x 50 mL) and with H₂O (2 x 50 mL). After the usual treatment of the organic extracts the obtained residue was purified by column chromatography (mobile phase: 10% AcOEt/Hexane), yielding the diester (567.2 mg, 80%).

Colorless oil, Rf: 0.80 (50% AcOEt/Hexane).

**¹H-NMR (CDCl₃, δ):** 3.72 (3H, s, -OCH₃); 3.72 (3H, s, -OCH₃); 3.52 (1H, t, *J*= 7.5 Hz, CH-2); 3.43 (2H, d, *J*= 5.7 Hz, CH₂-5); 2.01 (1H, q, *J*=6.7 Hz, CH-4); 1.72 (1H, q, *J*= 7.4 Hz, CH-3); 1.60 (1H, q, *J*= 6.5 Hz, CH-3); 0.89 (3H, s, CH₃-4); 0.87 (9H, s, CH₃-^{t}Bu); 0.02 (6H, s, (CH₃)₂-Si).

**¹³C-NMR (CDCl₃, δ):** 170.18 and 170.04 (C=O); 67.60 (CH₂-5); 52.42 (CH₃-O); 49.77 (CH-2); 33.70 (CH-4); 32.70 (CH₂-3); 25.87 (CH₃-^{t}Bu); 18.28 (C-Si); 16.58 (CH₃-4); -5.48 ((CH₃)₂-Si).

**MS (FAB⁺) [m/z, (%)]:** 319.17 ([M+1]⁺, 25); 261.10 ([M-^{t}Bu]⁺, 100); 187.13 ([M-OTBS]⁺, 39); 185.17 (26).

**HRMS (FAB⁺):** 319.1941 calculated for C₁₅H₃₁O₅Si and 319.1946 obtained.

### 8. Preparation of (S)-5-(tert-butyldimethylsilyloxy)-2-hydroxymethyl-3-methyl pentan-1-ol (23a) and (S)-5-(tert-butyldimethylsilyloxy)-3-methylpentan-1-ol.

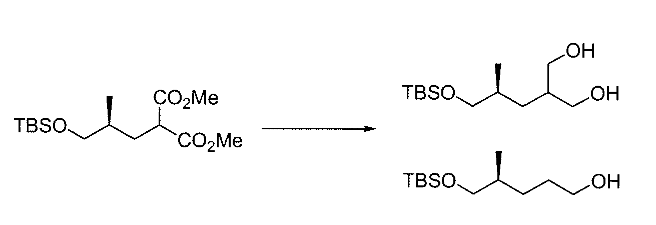

NaCl (291 mg; 5 mmol) and H₂O (103 µL) were added to a solution of diester (453 mg; 1.42 mmol) in DMSO (26 mL), and the mixture was heated at 160°C for 4 h. After heating 4h, AcOEt (50 mL) was added and it was washed with saturated NaCl solution (2x 50 mL) and with H2O (2 x 50 mL). The organic phase was dried, filtered and vacuum-concentrated. A 1.0 M solution of Dibal-H in hexane (4.3 mL; 4.3 mmol) was added to the raw product thus obtained dissolved in CH₂Cl₂ (7.5 mL) and cooled to -78°C, and left stirring at this temperature for 45 min. Then TBME (2 mL) and H₂O (0.3 mL) were added to the mixture and vigorous stirring was continued allowing to reach room temperature until the formation of a white gel, at this moment H₂O (0.3 mL) and a 4.0 M NaOH solution (0.3 mL) were added and the stirring was continued until the formation of a white precipitate, then Na₂SO₄ was added and the solids were separated by vacuum filtration, after the evaporation to dryness the filtered liquids a residue was obtained which was purified by column chromatography (mobile phase: 10% AcOEt/Hexane), obtaining the diol (36.5 mg, 10%), and the alcohol (140.3 mg, 50%).

Diol. Colorless liquid, Rf: 0.15 (50% AcOEt/Hexane).

**¹H-NMR (CDCl₃,** δ**):** 3.74 (2H, dt, *J=* 10.2 Hz, CH₂-1); 3.60 (2H, c, *J=* 10.4 Hz, CH₂-1'); 3.39 (2H, d, *J=* 6.1 Hz, CH₂-5); 1.85 (1H, m, CH-4); 1.65 (1H, sex., *J=* 6.7 Hz, CH-2); 1.33 (1H, m, CH-2); 0.96 (2H, m, CH-3); 0.87 (3H, d, *J=* 6.5 Hz, CH₃-4); 0.86 (9H, s,CH₃- ^{t}Bu); 0.02 (6H, s, CH₃-Si)

**¹³C-NMR (CDCl₃,** δ**):** 68.45 (CH₂-5); 66.97 (CH₂-1); 65.88 (CH₂-1'); 39.47 (CH-2); 33.19 (CH-4); 31.23 (CH₂-3); 25.89 (CH₃- ^{t}Bu); 18.29 (C-Si); 17.10 (CH₃-4); -5.43 (CH₃-Si).

**MS (FAB⁺) [m/z, (%)]:** 264.26 ([M+2]⁺, 20); 263.26 ([M+1]⁺, 100).

**HRMS (FAB⁺):** 263.2042 calculated for C₁₃O₃₁O₃Si and 263.2047 obtained.

Alcohol. Colorless liquid, Rf: 0.60 (50% AcOEt/Hexane).

**¹H-NMR (CDCl₃,** δ**):** 3.65 (2H, t, *J=* 6.8 Hz, CH₂-5); 3.41 (2H, dc, *J=* 10.2 Hz, *J=* 6.2 Hz, CH₂-1); 1.68-1.41 (4H, m, CH₂-2, CH₂-3); 1.13 (1H, m, CH-4); 0.88 (9H, s, CH₃-^{t}Bu); 0.88 (3H, d, *J=* 6.12 Hz, CH₃-4); 0.03 (6H, s, CH₃-Si)

**¹³C-NMR (CDCl₃,** δ**):** 68.24 (CH₂-5); 63.37 (CH₂-1); 35.54 (CH-4); 30.22 (CH₂-2); 29.22 (CH₂-3); 25.94 (CH₃- ^{t}Bu); 18.34 (C-Si); 16.74 (CH₃-4); -5.38 (CH₃-Si)

**MS (FAB⁺) [m/z, (%)]:** 233.26 ([M+1]⁺, 18); 215.28 ([M-OH]⁺, 100)

**HRMS (FAB⁺):** 233.1937 calculated for C₁₂H₂₉O₂Si and 233.1933 obtained.

### 9. Preparation of (tert-butyldiphenylsilyl) (S)-5-(tert-butyldimethylsilyloxy)-4-methylpentan-1-yl ether.

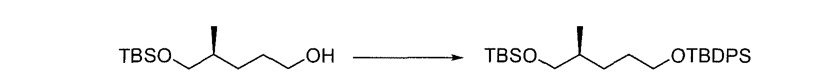

Imidazole (65 mg; 0.95 mmol), DMAP (c.c.), TBDPSCI (136 µL; 0.52 mmol) was added to a solution of alcohol (110 mg; 0.47 mmol) in DMF (1.8 mL) and it was stirred for 1 h. After that time H₂O was added, it was extracted with TBME (3 x 50 mL) and the pooled organic phases were washed with a saturated NaCl solution (75 mL), they were dried, filtered and vacuum-concentrated. A raw product was thus obtained which was purified by column chromatography (mobile phase: 4% AcOEt/Hexane), providing the protected compound (203.8 mg, 91%).

Colorless liquid, Rf: 0.90 (100% AcOEt).

**¹H-NMR (CDCl₃,** δ**):** 7.65 (4H, d, *J*= 7.24 Hz, CHₒ-Ph); 3.64 (2H, t, *J*= 7.10 Hz, CH₂-5); 3.38 (2H, dc, *J*= 9.75 Hz, *J*= 6.36 Hz, CH₂-1); 1.68-1.38 (4H, m, CH₂-3, CH₂-2); 1.07 (1H, m, CH-4); 1.03 (9H, s, ^{t}Bu-TBDPS); 0.88 (9H, s, ^{t}Bu-TBS); 0.84 (3H, d, *J*= 6.68 Hz, CH₃-4); 0.02 (6H, s, (CH₃)₂-Si).

**¹³C-NMR (CDCl₃, δ):** 135.57 (CHₒ-Ph); 134.17 (C-Ph); 129.46 (CHₚ-Ph); 127.55 (CHₘ-Ph); 68.37 (CH₂-5); 64.33 (CH₂-1); 35.57 (CH-4); 30.11 (CH₂-2); 29.34 (CH₂-3); 26.87 ((CH₃)₃-TBS); 25.96 (CH₃-TBDPS); 19.21 (C-TBDPS); 18.35 (C-TBS); 16.69 (CH₃-4); -5.36 ((CH₃)₂-Si).

**MS (FAB⁺) [m/z, (%)]:** 471.26 ([M+1]⁺, 12); 413.19 ([M-^{t}Bu]⁺, 18); 313.09 (23); 271.06 (46); 257.04 (29); 239.10 (21); 215.08 (46); 213.12 (22); 211.09 (31); 209.10 (79); 199.10 (49); 197.11 (100); 193.10 (31); 183.13 (29).

**HRMS (FAB⁺):** 471.3115 calculated for C₂₈H₄₇O₂Si₂ and 71.3108 obtained.

### 10. Preparation of (S)-5-(tert-butyldiphenylsilyloxy)-2-methylpentan-1-ol.

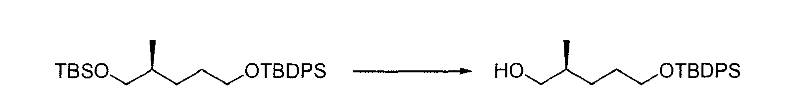

A solution of the diprotected compound (134 mg; 0.28 mmol) in an AcOH:THF:H₂O (3:1:1) mixture (10 mL) was maintained stirring at room temperature for 7 h 30 min. After that time a saturated K2CO3 solution was added dropwise until the bubbling stopped and it was extracted with AcOEt (3 x 20 mL). The pooled organic extracts were dried, filtered and vacuum-concentrated. A raw product was thus obtained which was purified by column chromatography (mobile phase: 10% AcOEt/Hexane), yielding the alcohol (95.8 mg, 94%).

Colorless liquid, Rf: 0.65 (50% AcOEt/Hexane).

**¹H-NMR (CDCl₃,** δ**):** 7.65 (4H, d, *J=* 7.11 Hz, CHₒ-Ph); 7.39 (6H, m, CH_{m, p}-Ph); 3.64 (2H, t, *J=* 6.40 Hz, CH₂-1); 3.48 (2H, dc, *J=* 10.40 Hz, *J=* 6.30 Hz, CH₂-5); 1.71-1.35 (4H, m, CH₂-3, CH₂-4); 1.15 (1H, m, CH-2); 1.05 (9H, s, CH₃-^{t}Bu); 0.90 (3H, d, *J=* 6.06 Hz, CH₃-2) **¹³C-NMR (CDCl₃,** δ**):** 135.56 (CHₒ-Ph); 134.05 (C-Ph); 129.51 (CHₚ-Ph); 127.58 (CHₘ-Ph); 68.25 (CH₂-1); 64.14 (CH₂-5); 35.46 (CH-2); 29.88 (CH₂-4); 29.18 (CH₂-3); 26.86 (CH₃-^{t}Bu); 19.19 (C-Si); 16.57 (CH₃-2)

**MS (FAB⁺) [m/z, (%)]:** 357.19 ([M+1]⁺, 24); 99.10 ([M-^{t}Bu]⁺, 14); 239.11 (16); 199.10 (100); 197.11 (42).

**HRMS (FAB⁺):** 357.2250 calculated for C₂₂H₃₃0₂Si and 357.2242 obtained.

### 11. Preparation of (S)-5-(tert-butyldiphenylsilyloxy)-2-methylpentanal .

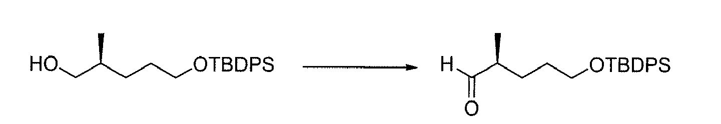

4Å molecular sieves (169 mg), NMO (66 mg; 0.56 mmol) and TPAP (c.c.) were added successively to a solution of alcohol (100 mg; 0.28 mmol) in CH₂Cl₂ (4.5 mL) and stirring was maintained for 30 min. After that time the solvent was eliminated under vacuum and the obtained residue was purified by column chromatography (mobile phase: 10% AcOEt/Hexane), providing the aldehyde (78.8 mg, 79%).

Colorless liquid, Rf: 0.75 (50% AcOEt/Hexane).

**¹H-NMR (CDCl₃, δ):** 9.60 (1H, s, CHO); 7.67 (4H, d, *J*= 7.24 Hz, CHₒ-Ph); 7.38 (6H, d, *J*= 7.10 Hz, CH_{p,m}-Ph); 3.68 (2H, m, CH₂-5); 2.50 (1H, m, CH-2); 1.75 (1H, m, CH-3); 1.69-1.48 (3H, m, CH₂-4, CH-3); 1.18 (3H, d, *J*= 6.82 Hz, CH₃-2); 1.05 (9H, s, CH₃-^{t}Bu).

**¹³C-NMR (CDCl₃, δ):** 128.71 (C=O); 135.55 (CHₒ-Ph); 133.92 (C-Ph); 129.60 (CHₚ-Ph); 127.61 (CHₘ-Ph); 63.56 (CH₂-5); 38.98 (CH-2); 29.81 (CH₂-3); 26.85 (CH₃-^{t}Bu); 19.19 (C-Si); 16.86 (CH₃-2)

**MS (FAB⁺) [m/z, (%)]:** 353.28 ([M-1]⁺, 15); 235.17 ([M-C₈H₆O]⁺, 52); 215.21 (22); 199.18 ([M-C₁₂H₁₁]⁺, 100); 183.21 (24); 181.20 (21).

**HRMS (FAB⁺):** 354.2015 calculated for C₂₂H₃₀O₂Si and 354.2019 obtained.

### 12. Preparation of (8S)-(4E,6Z)-11-(tert-butyldiphenylsilyloxy)-1-(3-furyl)-4,8-dimethylundecan-4,6-diene .

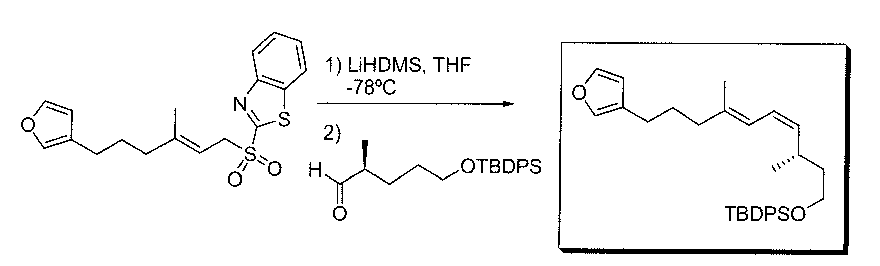

A 1.0 M solution of LiHDMS in THF (102 µL; 0.1 mmol) was added to a solution of the sulfone (39 mg; 0.11 mmol) in THF (0.4 mL) cooled to -78°C, observing that the solution acquired an intense orange color, after finishing the addition, the mixture was stirred under the same conditions for 1 h. Then a solution of aldehyde (26 mg; 0.74 mmol) in THF (0.4 mL) was added and stirring was extended for another 2 h. After that time, the cold bath was removed and saturated NH₄Cl solution was added, the aqueous phase was extracted with TBME (3 x 30 mL) and the pooled organic extracts were washed with a saturated NaCl solution (50 mL), they were dried, filtered and vacuum-concentrated. A raw product was thus obtained which was purified by column chromatography (mobile phase: 5% AcOEt/Hexane), yielding the diene [26 mg, 68%, **E;E/E;Z(1/4)].**

Colorless liquid, Rf: 0.82 (50% AcOEt/Hexane).

**¹H-NMR (CDCl₃,** δ**):** 7.66 (4H, d, *J=* 7.0 Hz, CHₒ-Ph); 7.37 (7H, m, CH_{m, p}-Ph, CH-5 furyl); 7.20 (1H, s, CH-2 furyl); 6.26 (1H, s, CH-4 furyl); 6.13 (1H, t, *J*= 11.1 Hz, CH-5); 6.04 (1 H, dd, *J*= 15.48 Hz, *J*= 10.60 Hz, CH-6); 5.11 (1H, t, *J*= 10.32 Hz, CH-7); 3.64 (2H, t, *J*= 6.54 Hz, CH₂-1); 2.60 (1H, m, CH-4); 2.40 (2H, t, *J*= 7.30 Hz, CH₂-11); 2.09 (2H, t, *J*= 7.50 Hz, CH₂-9); 1.84-1.62 (5H, m, CH₃-8, CH₂-10); 1.05 (9H, s, CH₃-^{t}Bu); 0.96 (3H, d, *J*= 6.80 Hz, CH₃-4).

**¹³C-NMR (CDCl₃,** δ**):** 142.64 (CH-5 furyl); 138.81 (CH-2 furyl); 138.05 (C-8); 136.36 (CH-5); 135.56 (CHₒ-Ph); 134.14 (C-Ph); 129.46 (CHₘ-Ph); 127.55 (CHₚ-Ph); 124.95 (C-3 furyl); 123.33 (CH-6); 120.41 (CH-7); 110.98 (CH-4 furyl); 64.06 (CH₂-1); 39.74 (CH₂-9); 33.65 (CH₂-3); 31.66 (CH-4); 30.49 (CH₂-2); 28.18 (CH₂-10); 26.87 (CH₃-^{t}Bu); 24.33 (CH₂-11); 21.34 (CH₃-4); 19.21 (C-Si); 16.27 (CH₃-8);

**MS (FAB⁺) [m/z, (%)]:** 501.32 ([M+1]⁺, 10); 199.11 (100); 197.13 (65); 183.15 (41); 181.14 (24).

**HRMS (FAB⁺):** 501.3189 calculated for C₃₃H₄₅O₂Si and 501.3169 obtained.

### EXAMPLE 7

### PREPARATION OF (6S)-(2E,7Z,9E)-1-(TERT-BUTYLCARBOXYL)-13-(3-FURYL)-2,6,10-TRIMETHYLTRIDECAN-2.7.9-TRIENE

### 1. Preparation of methyl (R)-3-(tert-butyldiphenylsilyloxy)-2-methylpropanoate.

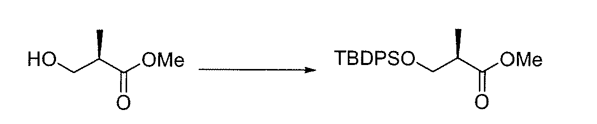

Imidazole (1.1 g; 16.24 mmol), DMAP (c.c.) and TBDPSCI (2.3 mL; 8.94 mmol) were added to a solution of alcohol (0.96 g; 8.12 mmol) in DMF (30 mL), and stirring was maintained for 12 h. After that time H₂O was added, the mixture was extracted with TBME (3 x 50 mL) and the pooled organic phases were washed with saturated NaCl solution (2 x 75 mL). After evaporating the solvent to dryness a raw product was obtained which was purified by column chromatography (mobile phase: 3% AcOEt/Hexane), obtaining the protected alcohol (3.2 g, 99%).

Colorless liquid, Rf: 0.30 (10% AcOEt/Hexane).

**¹H-NMR (CDCl₃, δ):** 7.65 (4H, dd, *J*= 7.70 Hz, *J=* 1.67 Hz, CHₒ Ph); 7.38 (6H, m, CH_{p,m}-Ph); 3.82 (1H, dd, *J*= 9.70 Hz, *J=* 6.96 Hz, CH-3); 3.72 (1H, dd, *J*= 9.80 Hz, *J*= 5.85 Hz, CH-3); 3.68 (3H, s, -OCH₃); 2.71 (1 H, sex., *J*= 6.76 Hz, CH-2); 1.15 (3H, d, *J*= 7.10 Hz, CH₃-2); 1.02 (9H, s, CH₃-^{t}Bu) **¹³C-NMR (CDCl₃, δ):** 175.37 (C=O); 135.56 (CHₒ-Ph); 133.53 (C-Ph); 129.64 (CHₚ-Ph); 127.64 (CHₘ-Ph); 65.90 (CH₂-3); 51.53 (CH₃O); 42.38 (CH-2); 26.69 (CH₃-^{t}Bu); 19.22 (C-Si); 13.46 (CH₃-2)

**MS (FAB⁺) [m/z, (%)]:** 357.16 ([M+1]⁺, 5); 300.08 (25); 299.31 ([M-^{t}Bu]⁺, 100); 279.11 (48); 213.10 (26).

**HRMS (FAB⁺):** 357.1886 calculated for C₂₁H₂₉O₃Si and 357.1897 obtained.

### 2. Preparation of (R)-3-(tert-butyldiphenylsilyloxy)-2-methylpropan-1-ol.

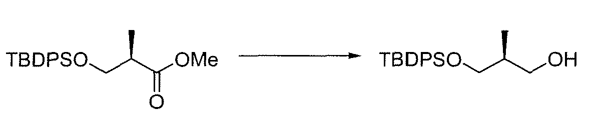

A 1.0 M solution of Dibal-H sin hexane (9.1 mL; 9.1 mmol) was added to a solution of the ester (1.6 g; 4.55 mmol) in CH₂Cl₂ (30 mL) and cooled to -78°C, and it was stirred for 2 h. After that time TBME (8.5 mL) and H₂O (1.3 mL) were added to the reaction mixture, maintaining vigorous stirring and allowing to reach room temperature until the formation of a white gel, in this moment H₂O (1.3 mL) and a 4.0 M NaOH solution (1.3 mL) were added and stirring was maintained until the formation of a white precipitate, then Na₂SO₄ was added and the solids were separated by vacuum filtration, after the evaporation to dryness of the filtered liquids, the residue thus obtained was purified by column chromatography (mobile phase: 10% AcOEt/Hexane), yielding the alcohol (1.56 g, 99%).

Colorless liquid, Rf: 0.15 (10% AcOEt/Hexane).

**¹H-NMR (CDCl₃,** δ**):** 7.67 (4H, dd, *J=* 7.79 Hz, *J=* 1.59 Hz, CHₒ-Ph); 7.41 (6H, m, CHₚ-Ph); 3.72 (1H, dd, *J=* 10.01 Hz, *J=* 4.45 Hz, CH-3); 3.67 (2H, b.s., CH₂-1); 3.59 (1H, dd, *J=* 10.01 Hz, *J=* 7.63 Hz, CH-3); 2.49 (1H, s.a., -OH); 1.99 (1 H, sex., *J=* 6.20 Hz, CH-2); 1.05 (9H, s, CH₃-^{t}Bu); 0.82 (3H, d, *J=* 6.99 Hz, CH₃-2)

**¹³C-NMR (CDCl₃,** δ**):** 135.58 (CHₒ-Ph); 133.15 (C-Ph); 129.78 (CHₘ-Ph); 127.75 (CHₚ-Ph); 68.74 (CH₂-3); 67.70 (CH₂-1); 37.28 (CH-2); 26.83 (CH₃-^{t}Bu); 19.13 (C-Si); 13.14 (CH₃-2).

**MS (FAB⁺) [m/z, (%)]:**329.11 ([M+1]⁺, 43); 311.11 ([M-OH]⁺, 21); 271.05 ([M-^{t}Bu]⁺, 38); 199.10 (100); 197.12 (30); 193.12 (26).

**HRMS (FAB⁺):** 329.1937 calculated for C₂₀H₂₉O₂Si and 329.1935 obtained.

### 3. Preparation of (R)-3-(tert-butyldiphenylsilyloxy)-2-methyl-iodopropane .

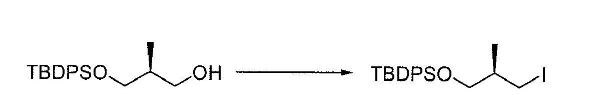

PPh₃ (2.4 g; 9.28 mmol) and imidazole (1.6 g; 23.07 mmol) were added to a solution of the alcohol (2.5 g; 7.69 mmol) in THF (33 mL) and the mixture was stirred until completely dissolved, then it was cooled to -20°C and iodine (2.2 g; 8.46 mmol) was added, the appearance of an orange color being observed, stirring was maintained under the same conditions for 15 min, and then the cold bath was removed allowing the mixture to reach room temperature for 30 min, after that time and with the flask immersed in an ice bath a saturated NaHCO3 solution (50 mL) was added, the formation of a white precipitate then being observed and the solution acquired a transparent yellow color. The pooled organic phases were washed with a 10% Na₂SO₃ solution (100 mL) and with H₂O (100 mL), they were dried, filtered and vacuum-concentrated. The raw product thus obtained was purified by column chromatography (mobile phase: 10% AcOEt/Hexane), providing iodide (3 g, 90%).

Yellow liquid, Rf: 0.85 (50% AcOEt/Hexane).

**¹H-NMR (CDCl₃, δ):** 7.68 (4H, m, CHₒ-Ph); 7.43 (6H, m, CH_{p,m}-Ph); 3.60 (1H, dd, *J*= 10.15 Hz, *J*= 4.87 Hz, CH-1); 3.48 (1H, dd, *J*= 10.02 Hz, *J*= 6.96 Hz, CH-1); 3.41 (1H, dd, *J*= 9.50 Hz, *J*= 5.15 Hz, CH-3); 3.34 (1H, dd, *J*= 9.50 Hz, *J*= 5.80 Hz, CH-3); 1.74 (1H ,sex., *J*= 5.90 Hz, CH-2); 1.08 (9H, s, CH₃-^{t}Bu); 0.98 (3H, d, *J*= 6.68 Hz, CH₃-2)

**¹³C-NMR (CDCl₃, δ):** 135.63 (CHₒ-Ph); 133.58 (C-Ph); 129.66 (CHₘ-Ph); 127.66 (CHₚ-Ph); 67.33 (CH₂-1); 37.53 (CH-2); 26.84 (CH₃-^{t}Bu); 19.29 (C-Si); 17.31 (CH₂-3); 13.51 (CH₃-2)

**MS (FAB⁺) [m/z, (%)]:** 439.05 ([M+1]⁺, 3); 380.97 ([M-^{t}Bu]⁺, 41); 308.91 (100); 246.92 (34); 199.09 (26); 197.11 (38).

**HRMS (FAB⁺):** 439.0954 calculated for C₂₀H₂₈OSil and 439.0967 obtained.

### 4. Preparation of dimethyl (R)-4-(tert-butyldiphenylsilyloxy)-3-methylbutane-1,1-dicarboxylate.

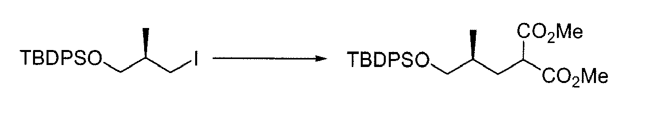

Dimethyl malonate (1.6 mL; 13.6 mmol) was added to a 60% NaH solution (0.55 g; 13.6 mmol) in DMF (1.5 mL) and THF (10 mL), the formation of a white foam being observed, then a solution of the iodide (3 g; 6.9 mmol) in THF (20 mL) was added and the stirred mixture was heated under reflux for 18 h. After that time it was allowed to reach room temperature, AcOEt (100 mL) was added, the organic layer was washed with saturated NaCl solution (2 x 100 mL) and with H₂O (2 x 100 mL), after evaporating the solvent under vacuum the raw product thus obtained was purified by column chromatography (mobile phase: 6% AcOEt/Hexane), obtaining the diester (2.76 g, 91%).

Colorless oil, Rf: 0.50 (30% AcOEt/Hexane).

**¹H-NMR (CDCl₃, δ):** 7.64 (4H, dd, *J*= 7.80 Hz, *J*= 1.53 Hz, CHₒ-Ph); 7.39 (6H, m, CH_{p,m}-Ph); 3.71 (6H, s,-OCH₃); 3.52 (1 H, t, *J*= 7.66 Hz, CH-2); 3.49 (2H, d, *J*= 5.71 Hz, CH₂-5); 2.11 (1H, m, CH-3); 1.76 (1H, m, CH-3); 1.67 (1H, m, CH-4); 1.05 (9H, s, CH₃-^{t}Bu); 0.92 (3H, d, *J*= 6.54 Hz, CH₃-4) **¹³C-NMR (CDCl₃, δ):** 170.14 (C=O); 169.98 (C=O); 135.60 (CHₒ-Ph); 133.69 (C-Ph); 129.58 (CHₘ-Ph); 127.62 (CHₚ-Ph); 68.37 (CH₂-5); 52.43 (CH₃O); 49.72 (CH-2); 33.71 (CH-4); 32.60 (CH₂-3); 26.82 (CH₃-^{t}Bu); 19.26 (C-Si); 16.64 (CH₃-4)

**MS (FAB⁺) [m/z, (%)]:** 443.33 ([M+1]⁺, 8); 386.23 (29); 385.22 ([M-^{t}Bu]⁺, 100); 365.24 (36); 213.17 (40).

**HRMS (FAB⁺):** 443.2254 calculated for C₂₅H₃₅O₅Si and 443.2254 obtained.

### 5. Preparation of (S)-5-(tert-butyldiphenylsilyloxy)-2-hydroxymethyl-3-methylpentan-1-ol and (S)-5-(tert-butyldiphenylsilyloxy)-3-methylpentan-1-ol.

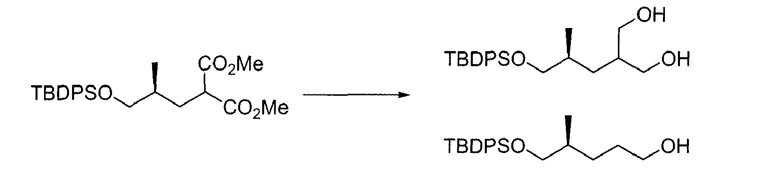

NaCl (1.2 g; 20.21 mmol) and H₂O (415.5 mL) were added to a solution of the diester (2.6 g; 5.77 mmol) in DMSO (120 mL), and the mixture was heated at 160°C with stirring. Its important to emphasize the difficulty of following the reaction por t.l.c., therefore even without the certainty of the reaction having ended, after 4h and 30 min the mixture was allowed to reach room temperature, AcOEt (100 mL) was added and it was washed with saturated NaCl solution (2 x 100 mL) and with H₂O (2 x 100 mL). The organic phase was dried, filtered and vacuum-concentrated. The residue thus obtained dissolved in Et₂O (44 mL) was placed in a flask provided with condenser and immersed in an ice bath. LiAlH₄ (0.64 g; 16.95 mmol) was added in small portions to this solution, observing that the mixture was under reflux. After finishing the addition the cold bath was removed and the reaction mass was maintained stirring at room temperature for 30 min, after that time and with the flask immersed in an ice bath H₂O was added dropwise until the end of the bubbling was observed, in this moment the mixture was maintained stirring vigorously for 30 min and Na₂SO₄ was added, the solids were separated by filtration and the filtered liquids were concentrated to dryness, the raw product thus obtained, was purified by column chromatography (mobile phase: 10% AcOEt/Hexane), yielding the diol (300 mg, 16%) and the alcohol (1.47 g, 73%).

Diol. Colorless liquid, Rf: 0.15 (50% AcOEt/Hexane).

**¹H-NMR (CDCl₃,** δ**): ):** 7.64 (4H, d, *J*= 6.84 Hz, CHₒ-Ph); 7.39 (6H, m, CH_{p,m}-Ph); 3.75 (2H, dt, *J*= 10.81 Hz, *J*= 3.49 Hz, CH₂-1); 3.59 (2H, m, CH₂-1'); 3.47 (2H, d, *J*= 5.72 Hz, CH₂-5); 1.92 (1H, s, -OH); 1.81 (1H, s, -OH); 1.69 (1H, sex., *J*= 6.64 Hz, CH-4); 1.35 (1H, q, *J*= 6.80 Hz, CH-2); 1.05 (9H, s, CH₃-^{t}Bu); 0.98 (2H, dd, *J*= 13.99 Hz, *J*= 6.84 Hz, CH₂-3); 0.92 (3H, d, *J*= 6.76 Hz, CH₃-4).

**¹³C-NMR (CDCl₃,** δ**):** 135.63 (CH₀-Ph); 133.87 (C-Ph); 129.59 (CHₘ-Ph); 127.61 (CHₚ-Ph); 68.93 (CH₂-5); 67.27 (CH₂-1); 66.47 (CH₂-1'); 39.44 (CH-2); 33.26 (CH-4); 31.19 (CH₂-3); 26.69 (CH₃-^{t}Bu); 19.28 (C-Si); 17.18 (CH₃-4).

**MS (FAB⁺) [m/z**, **(%)]:** 387.23 ([M+1]⁺, 14); 199.11 (100); 197.13 (52).

**HRMS (FAB⁺):** 387.2355 calculated for C₂₃H₃₅O₃Si and 387.2351 obtained.

Alcohol. Colorless liquid, Rf: 0.60 (50% AcOEt/Hexane).

**¹H-NMR (CDCl₃,** δ**):** 7.66 (4H, dd, *J*= 7.95 Hz, *J*= 1.59 Hz, CHₒ-Ph); 7.39 (6H, m, CH_{p,m}-Ph); 3.59 (2H, t, *J*= 6.68 Hz, CH₂-1); 3.51 (1H, dd, *J*= 9.86 Hz, *J*= 5.88 Hz, CH-5); 3.46 (1H, dd, *J*= 9.86 Hz, *J*= 6.20 Hz, CH-5); 1.66 (1H, sex., *J*= 6.36 Hz, CH-4); 1.61-1.39 (4H, m, -OH, CH₂-2, CH-3); 1.16 (1H, m, CH-3); 1.05 (9H, s, CH₃-^{t}Bu); 0.92 (3H, d, *J*= 6.68 Hz, CH₃-4)

**¹³C-NMR (CDCl₃, δ):** 135.61 (CHₒ-Ph); 134.03 (C-Ph); 129.50 (CHₘ-Ph); 127.57 (CHₚ-Ph); 68.76 (CH₂-5); 63.33 (CH₂-1); 35.53 (CH-4); 30.19 (CH₂-2); 29.15 (CH₂-3); 26.88 (CH₃-^{t}Bu); 19.31 (C-Si); 16.83 (CH₃-4)

**MS (FAB⁺) [m/z, (%)]:** 357.15 ([M+1]⁺, 14); 299.07 ([M-^{t}Bu]⁺, 14); 199.09 (100); 197.11 (42).

**HRMS (FAB⁺):** 357.2250 calculated for C₂₂H₃₃O₂Si and 357.2264 obtained.

### 6. Preparation of (R)-3-(tert-butyldiphenylsilyloxy)-2-methyl propanal.

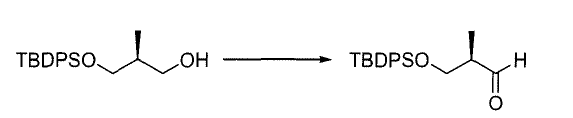

DMSO solution (1.6 mL; 11.86 mmol) in CH₂Cl₂ (8.5 mL) was added dropwise to a solution of 2.0 M oxalyl chloride in CH₂Cl₂ (5.9 mL; 11.83 mmol) in CH₂Cl₂ (4.8 mL) and cooled to -78°C, the occurrence of bubbling being observed. After stirring for 20 min under the same conditions, the alcohol (1.9 g; 5.93 mmol) dissolved in CH₂Cl₂ (8.5 mL) was added and stirring was extended for 20 min. Finally Et₃N (5.9 mL) was added and the cold bath was removed, maintaining the stirring for another 30 min. After that time H₂O (20 mL) was added and it was extracted with AcOEt (3 x 20 mL). After the usual treatment of the organic extracts, the aldehyde (2.01 g, 99%) was obtained.

White solid, Pf: 69-71°C, Rf: 0.55 (30% AcOEt/Hexane).

**¹H-NMR (CDCl₃, δ):** 9.77 (1H, d, *J*= 1.53 Hz, CH-1); 7.65 (4H, dd, *J*= 7.94 Hz, *J=* 1.40 Hz, CHₒ-Ph); 7.41 (6H, m, CH_{m,p}-Ph); 3.91 (1H, dd, *J*= 10.30 Hz, *J*= 5.01 Hz, CH-3); 3.85 (1H, dd, *J*= 10.40 Hz, *J*= 6.30 Hz, CH-3); 2.57 (1H, tc, *J*= 6.68 Hz, *J*= 1.58 Hz, CH-2 ); 1.10 (3H, d, *J*= 7.10 Hz, CH₃-2); 1.05 (9H, s, CH₃-^{t}Bu).

**¹³C-NMR (CDCl₃, δ):** 204.41 (C=O); 135.57 (CHₒ-Ph); 133.17 (C-Ph); 129.79 (CHₘ-Ph); 127.74 (CHₚ-Ph); 64.13 (CH₂-3); 48.40 (CH-2); 26.75 (CH₃-^{t}Bu); 19.23 (C-Si); 10.29 (CH₃-2).

**MS (FAB⁺) [m/z, (%)]:** 325.12 ([M-1]⁺, 9); 309.13 ([M-OH]⁺, 24); 269.08 ([M-^{t}Bu]⁺, 100).

**HRMS (FAB⁺):** 327.1780 calculated for C₂₀H₂₇O₂Si and 327.1773 obtained.

### 7. Preparation of ethyl (S)-(E)-5-(tert-butyldiphenylsilyloxy)-4-methylpent-2-enoate.

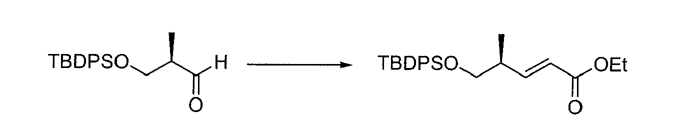

The aldehyde (2.2 g; 6.59 mmol) dissolved in THF (42 mL) was placed in a flask provided with stirring and condenser. (Ethoxycarbony-Imethylene)triphenylphosphorane (2.7 g; 7.26 mmol) was added to this solution and the mixture was heated under reflux for 3 h. After that time and once the reaction mass reached room temperature the solvent was vacuum eliminated. The raw product thus obtained was purified by column chromatography (mobile phase: 5% AcOEt/Hexane), providing the ester (2.3 g, 88%).

Yellow oil, Rf 0.40 (10% AcOEt/Hexane).

**¹H-NMR (CDCl₃, δ):** 7.64 (4H, m, CHₒ-Ph); 7.39 (6H, m, CH_{p,m}-Ph); 6.95 (1H, dd, *J*= 15.89 Hz, *J*= 1.27 Hz, CH-3); 5.83 (1H, dd, *J*= 15.74 Hz, *J*= 1.27 Hz, CH-2 ); 4.19 (2H, c, *J*= 7.10 Hz, CH₂-OEt); 3.58 (2H, dd, *J*= 6.36 Hz, *J*= 2.54 Hz, CH₂-5); 2.55 (1H, q, *J*= 6.75 Hz, CH-4); 1.28 (3H, t, *J*= 7.15 Hz, CH₃-OEt); 1.07 (3H, d, *J*= 6.83 Hz, CH₃-4) 1.04 (9H, s, CH₃-^{t}Bu).

**¹³C-NMR (CDCl₃, δ):** 166.69 (C=O); 151.30 (CH-3); 135.60 (CHₒ-Ph); 133.58 (C-Ph); 129.64 (CHₘ-Ph); 127.65 (CHₚ-Ph); 67.55 (CH₂-5); 60.16 (CH₃-OEt); 39.09 (CH-4); 26.81 (CH₃-^{t}Bu); 19.27 (C-Si); 15.57 (CH₃-4); 14.27 (CH₃-OEt).

**MS (FAB⁺) [m/z, (%)]:** 395.22 ([M-1]⁺, 12); 339.14 ([M- ^{t}Bu]⁺, 98); 319.16 ([M-C₂H₆O₂]⁺, 100).

**HRMS (FAB⁺):** 395.2042 calculated for C₂₄H₃₁O₃Si and 395.2027 obtained.

### 8. Preparation of ethyl (S)-5-(tert-butyldiphenylsilyloxy)-4-methylpentanoate.

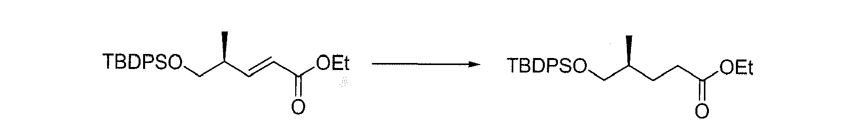

A dispersion of the alkene (1.1 g; 2.76 mmol) and Pd/C (10%) (75 mg) in MeOH (9 mL) was stirred vigorously and under H₂ atmosphere for 16 h. Then the reaction mixture was filtered through celite to separate the catalyst and the liquids were concentrated to dryness, obtaining the reduced ester (1.1 g, 99%).

Colorless liquid, Rf: 0.65 (30% AcOEt/Hexane).

**¹H-NMR (CDCl₃, δ):** 7.65 (4H, dd, *J*= 7.87 Hz, *J=* 1.60 Hz, CHₒ-Ph); 7.39 (6H, m, CH_{p,m}-Ph); 4.10 (2H, c, *J*= 7.24 Hz, CH₂-OEt); 3.49 (1 H, dd, *J*= 9.88 Hz, *J*= 5.98 Hz, CH-5); 3.45 (1H, dd, *J*= 10.02 Hz, *J*= 5.84 Hz, CH-5); 2.28 ( 2H, m, CH₂-2); 1.80 (1H, m,CH-3); 1.67(1H, sex., *J*= 6.49 Hz, CH-4); 1.47 ( 1H, m, CH-3); 1.23 ( 3H, t, *J*= 7.25 Hz, CH₃-OEt); 1.04 (9H, s, CH₃-^{t}Bu); 0.92 (3H, d, *J*= 6.83 Hz, CH₃-4).

**¹³C-NMR (CDCl₃, δ):** 173.31 (C=O); 135.60 (CHₒ-Ph); 133.92 (C-Ph); 129.53 (CHₘ-Ph); 127.59 (CHₚ-Ph); 68.47 (CH₂-5); 60.17 (CH₃-OEt); 35.30 (CH-4); 32.11 (CH₂-2); 28.45 (CH₃-^{t}Bu); 19.36 (C-Si); 16.52 (CH₃-4); 14.23 (CH₃-OEt).

**MS (FAB⁺) [m/z, (%)]:** 399.15 ([M-1]⁺, 6); 341.11 ([M-^{t}Bu]⁺, 100).

**HRMS (FAB⁺):** 399.2355 calculated for C₂₄H₃₅O₃Si and 399.2343 obtained.

### 9. Preparation of (S)-5-(tert-butyldiphenylsilyloxy)-4-methylpentanal.

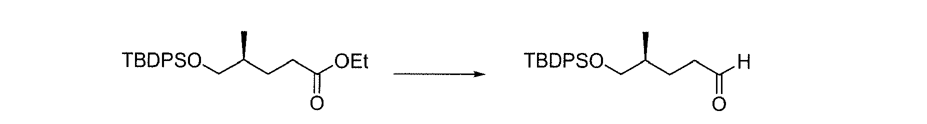

A 1.0 M solution of Dibal-H in hexane (3.4 mL; 3.4 mmol) dissolved in CH₂Cl₂ (1 mL) was added dropwise to a solution of the ester (1.2 g; 3.1 mmol) in CH₂Cl₂ (19 mL) and cooled to -78°C. After finishing the addition stirring was maintained for 45 min under the same conditions. Then a mixture formed by a saturated NaCl solution (5 mL), a saturated Na/K tartrate (10 mL) solution, H₂O (5 mL) and Et₂O (20 mL) was added to the reaction mixture and was vigorously stirred, until the disappearance of the turbidity. Once the solution reached room temperature it was extracted with Et₂O (3 x 50 mL) and the pooled organic phases were dried, filtered and vacuum-concentrated, obtaining the virtually pure aldehyde (1.06 g, 97%).

Colorless liquid, Rf: 0.20 (10% AcOEt/Hexane).

**¹H-NMR (CDCl₃,** δ**):** 9.75 (1 H, s, CHO); 7.67 (4H, d, *J=* 6.22 Hz, CHₒ-Ph); 7.41 (6H, m, CH_{p,m}-Ph); 3.51 (1H, d, *J*= 5.93 Hz, CH-5); 2.40 ( 2H, dd, *J*= 14.13 Hz, *J*= 7.06 Hz, CH₂-2); 1.87 (1H, sex., *J*= 7.02 Hz, CH-3); 1.70 (1H, sex., *J*= 6.28 Hz, CH-4); 1.5 ( 1 H, sex., *J*= 7.12 Hz, CH-3); 1.08 (9H, s, CH₃-^{t}Bu); 0.93 (3H, d, *J*= 6.78 Hz, CH₃-4).

**¹³C-NMR (CDCl₃,** δ**):** 202.77 (CHO); 135.59 (CHₒ-Ph); 133.75 (C-Ph); 129.58 (CHₘ-Ph); 127.61 (CHₚ-Ph); 66.28 (CH₂-5); 41.79 (CH₂-2); 35.34 (CH-4); 26.86 (CH₃-^{t}Bu); 25.36 (CH₂-3); 19.27 (C-^{t}Bu); 16.59 (CH₃-4).

**MS (FAB⁺) [m/z, (%)]:** 355.2049 ([M+1]⁺, 4); 297.01 ([M-^{t}Bu]⁺, 100).

**HRMS (FAB⁺):** 354.2015 calculated for C₂₂H₃₀O₂Si and 354.3506 obtained.

### 10. Preparation of ethyl (6S)-(2E)-7-(tert-butyldiphenylsilyloxy)-2.6-dimethylhept-2-enoate.

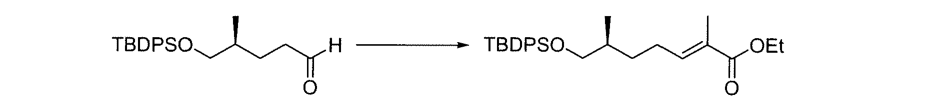

(1-ethoxycarbonylethylidene)triphenylphosphorane (1.2 g; 3.1 mmol) was added to a solution of the aldehyde (1.1 g; 3.1 mmol) in THF (20 mL) and the well stirred mixture was maintained under reflux for 15 h. After that time and once the reaction mass reached room temperature the solvent was eliminated under reduced pressure. The obtained residue was purified by column chromatography (mobile phase: 3% AcOEt/Hexane), yielding the ester (1.29 g, 97%).

Transparent oil. Rf: 0.45 (10% AcOEt/Hexane).

**¹H-NMR (CDCl₃, δ):** 7.65 ( 4H, d, *J=* 7.64 Hz, CHₒ-Ph ); 7.39 ( 6H, m, CH_{p,m}-Ph); 6.73 (1H, t, *J=* 7.20 Hz, CH-3); 4.18 ( 2H, c, *J=* 7.01 Hz, CH₂-OEt); 3.48 ( 2H, dd, *J=* 5.43 Hz, *J=* 3.90 Hz, CH₂-7 ); 2.14 ( 1H, q, *J=* 7.38 Hz, CH-6 ); 1.27 ( 5H, m, CH₃-OEt, CH₂-5 ); 1.04 ( 9H, s, CH₃-^{t}Bu); 0.93 ( 3H, d, *J=* 6.54 Hz, CH₃-6).

**¹³C-NMR (CDCl₃, δ):** 168.28 ( C=O); 142.40 ( CH-3); 135.61 (CHₒ-Ph); 133.97 (C-Ph); 129.57(CHₚ-Ph); 127.68 (C-2); 127.58 (CHₘ-Ph); 68.52 (CH₂-7); 60.32 (CH₂-OEt); 35.43 (CH-6); 31.98 (CH₂-5); 26.88 (CH₃-^{t}Bu); 26.20 (CH₂-4), 19.31 (C-Si); 16.70 (CH₃-6); 14.29 (CH₃-OEt); 12.30 (CH₃-2).

**MS (FAB⁺) [m/z, (%)]:** 381.16 ([M-^{t}Bu]⁺, 94); 199.08 ([M-C₁₅H₂₇O₂]⁺, 100).

**HRMS (FAB⁺):** 381.1886 calculated for C₂₃H₂₉O₃Si and 381.1894 obtained.

### 11. Preparation of (6S)-(2E)-7-(tert-butyldiphenylsilyloxy)-2,6-dimethylhept-2-en-1-ol.

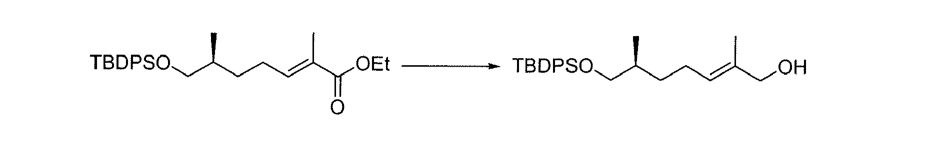

A 1.0 M solution of Dibal-H in hexane (5.7 mL; 5.7 mmol) was added to a solution of the ester (0.83 g; 1.89 mmol) in CH₂Cl₂ (12 mL) and cooled to -78°C, and left stirring for 30 min. After that time TBME (2.6 mL) and H₂O (0.4 mL) were added to the reaction mixture and it was maintained with vigorous stirring allowing it to reach room temperature until the formation of a white gel, in this moment H₂O (0.4 mL) and 4.0 M NaOH solution (0.4 mL) was added and stirring was extended until the formation of a white precipitate, then Na₂SO₄ was added and the solids were vacuum filtered, the filtered liquids were concentrated to dryness under reduced pressure. The raw product thus obtained was purified by column chromatography (mobile phase: 8% AcOEt/Hexane), providing the allyl alcohol (0.72 g, 96%).

Colorless liquid, Rf: 0.60 (30% AcOEt/Hexane).

**¹H-NMR (CDCl₃, δ):** 7.66 ( 4H, dd, *J*= 7.79 Hz, *J*= 1.53 Hz, CHₒ Ph ); 7.39 ( 6H, m, CH_{p,m}-Ph); 5.73 ( 1H, t, *J*= 7.00 Hz, CH-3); 3.98 ( 2H, s, CH₂-1 ); 3.52 ( 1H, dd, *J*= 9.89 Hz, *J*= 5.60 Hz, CH-7 ); 3.46 (1H, dd, *J*= 9.89 Hz, *J*= 6.13 Hz, CH-7); 2.02 ( 2H, sept., *J*= 7.86 Hz, CH₂-4 ); 1.67 ( 1H, m, CH-5 ); 1.64 (3H, s, CH₃-2); 1.51 (1H, m CH-5); 1.37( 1H, b.s., -OH); 1.18 (1H, m, CH-5); 1.05 ( 9H, s, CH₃-^{t}Bu ); 0.94 ( 3H, d, *J*= 6.68 Hz, CH₃-6).

**¹³C-NMR (CDCl₃, δ):** 135.61 (CHₒ-Ph); 134.57 (C-2); 134.07 (C-Ph); 129.48( CHₚ-Ph); 127.55 (CHₘ-Ph); 126.63 (CH-3); 69.54 (CH₂-7); 68.73 (CH₂-1 ); 35.36 (CH-6); 32.90 (CH₂-5); 26.88 (CH₃-^{t}Bu); 25.26 (CH₂-4), 19.31 (C-Si); 16.79 (CH₃-6); 13.26 (CH₃-2).

**MS (FAB⁺) [m/z, (%)]:** 321.12 ([M-^{t}Bu-OH]⁺, 4); 199.08 ([M-C₁₃H₂₆O]⁺, 100).

**HRMS (FAB⁺):** 321.1675 calculated for C₂₁H₂₅OSi and 321.1664 obtained.

### 12. Preparation of (6S)-(2E)-7-(tert-butyidiphenylsilyloxy)-2,6-dimethylhept-2-enyl pivaloate.

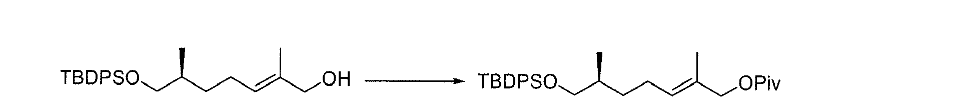

Pivaloyl chloride (2 mL; 16.2 mmol) was added to a solution of allyl alcohol (1.3 g; 3.24 mmol) and DMAP (conc.) in pyridine (52 mL) cooled to 0°C. After finishing the addition the cold bath was removed and the mixture was stirred for 4 h. After that time and with the flask immersed in an ice bath a 10% HCI acid solution (52 mL) was added, it was extracted with AcOEt (2 x 100 mL) and the pooled organic phases were first washed with H₂O (2 x 150 mL), then with a 10% CuSO₄ solution (3 x 150 mL) and finally with H₂O (150 mL). After the usual treatment of the organic phases a raw product was obtained which was purified by column chromatography (mobile phase: 5% AcOEbHexane), providing the pivaloate (1.6 g, 99%).

Colorless liquid, Rf: 63 (30% AcOEt/Hexane).

**¹H-NMR (CDCl₃, δ):** 7.65 (4H, dd, *J*= 7.66 Hz, *J*= 1.39 Hz, CHₒ-Ph); 7.39 (6H, m, CH_{p,m}-Ph); 5.40 (1 H, t, *J*= 7.10 Hz, CH-3); 4.43 (2H, s, CH₂-1); 3.50 (1 H, dd, *J*= 9.88 Hz, *J*= 5.71 Hz, CH-7); 3.45 (1H, dd, *J*= 9.88 Hz, *J*= 5.99 Hz, CH-7); 2.01 (2H, sept., *J*= 7.94 Hz, CH₂-4); 1.69-1.45 (2H, m, CH₂-5); 1.60 (3H, s, CH₃-2); 1.18 (1H, m, CH-6); 1.20 (9H, s, ^{t}Bu-Piv); 1.04 (9H, s, ^{t}Bu-TBDPS); 0.92 (3H, d, *J*= 6.68 Hz, CH₃-6).

**¹³C-NMR (CDCl₃, δ):** 178.39 (C=O); 135.61 (CHₒ-Ph); 134.05 (C-Ph); 130.12 (C-2); 129.42 (CHₘ-Ph); 129.26 (CH-3); 127.56 (CHₚ-Ph); 69.98 (CH₂-1); 68.70 (CH₂-7); 38.85 (C-Piv); 35.37 (CH-6); 32.75 (CH₂-5); 27.22 (CH₃-Piv); 27.04 (CH₃-TBDPS); 25.14 (CH₂-4); 19.32 (C-Si); 16.76 (CH₃-6); 13.75 (CH₃-2).

**MS (FAB⁺) [m/z, (%)]:** 481.23 ([M+1]⁺, 6); 379.19 (20); 284.07 (25); 283.07 (100); 263.11 (30); 239.10 (20); 223.09 (41); 199.10 (67); 197.12 (41).

**HRMS (FAB⁺):** 481.3138 calculated for C₃₀H₄₅O₃Si and 481.3152 obtained.

### 13. Preparation of (6S)-(2E)-7-hydroxy-2,6-dimethylhept-2-enyl pivalate.

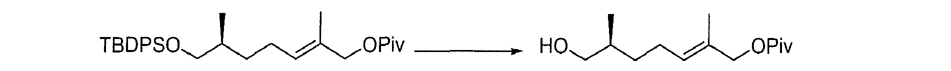

A 1.0 M solution of TBAF in THF (0.6 mL; 0.6 mmol) was added to a solution of the pivaloate (260 mg; 0.54 mmol) in THF (8 mL). After finishing the addition the mixture was maintained stirring at room temperature for 2 h and was left stirring for 2 h. After that time and with the flask immersed in an ice bath a saturated NaHCO3 solution was added, the white aqueous solution was extracted with CH₂Cl₂ (3 x 10 mL). The pooled organic extracts were dried, filtered and vacuum-concentrated. A residue was thus obtained which was purified by column chromatography (mobile phase: 10% AcOEt/Hexane), obtaining the alcohol (120.7 mg, 93%).

Colorless liquid, Rf: 0.25 (30% AcOEt/Hexane).

**¹H-NMR (CDCl₃,** δ**):** 5.42 (1H, t, *J=* 6.98 Hz, CH-3); 4.43 (2H, s, CH₂-1); 3.50 (1H, dd, *J=* 10.48 Hz, *J=* 5.91 Hz, CH-7); 3.43 (1H, dd, *J=* 10.48 Hz, *J=* 6.31 Hz, CH-7); 2.07 (2H, sex., *J=* 7.77 Hz, CH₂-4); 1.69-1.39 (3H, m, CH₂-5, CH-6); 1.63 (3H, s, CH₃-2); 1.20 (9H, s, ^{t}Bu-Piv); 0.92 (3H, d, *J=* 6.72 Hz, CH₃-6).

**¹³C-NMR (CDCl₃, δ):** 178.40 (C=O); 130.35 (C-2); 128.91 (CH-3); 69.91 (CH₂-1); 68.14 (CH₂-7); 38.85 (C-Piv); 35.37 (CH-6); 32.68 (CH₂-5); 27.22 (CH₃-Piv); 25.09 (CH₂-4); 16.46 (CH₃-6); 13.76 (CH₃-2).

**MS (FAB⁺) [m/z, (%)]:** 243.19 ([M+1]⁺ 15); 101.26 (29); 85.05 (100).

**HRMS (FAB⁺):** 243.1960 calculated for C₁₄H₂₇O₃ and 243.1856 obtained.

### 14. Preparation of (6S)-(2E)-2,6-dimethyl-7-oxohept-2-enyl pivalate

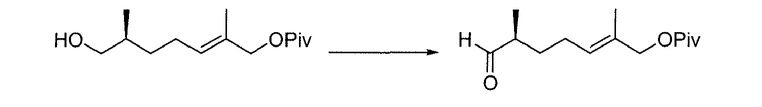

4Å molecular sieves (251 mg), NMO (223 mg; 1.90 mmol) and TPAP (c.c.) were added successively to a solution of the alcohol (231 mg; 0.95 mmol) in CH₂Cl₂ (9 mL). Then the reaction mixture was stirred at room temperature for 5 h, after that time the solvent was eliminated under vacuum and the resulting residue was purified by column chromatography (mobile phase: 5% AcOEt/Hexane), providing the aldehyde (176 mg, 77%).

Colorless liquid, Rf: 0.60 (30% AcOEt/Hexane).

**¹H-NMR (CDCl₃, δ):** 9.61 (1H, d, *J=* 1.95 Hz, CHO); 5.40 (1H, dt, *J=* 7.10 Hz, *J=* 1.25 Hz, CH-5); 4.43 (2H, s, CH₂-7); 2.34 (1H, dsex, *J=* 6.96 Hz, *J=* 1.90 Hz, CH-2); 2.09 (2H, c, *J=* 7.38 Hz, CH₂-4); 1.79 (1H, m, CH-3); 1.62 (3H, s, CH₃-6); 1.20 (9H, s, ^{t}Bu-Piv); 1.10 (3H, d, *J=* 7.10 Hz, CH₃-2).

**¹³C-NMR (CDCl₃,** δ**):** 204.81 (CHO); 178.31 (C=O); 131.48 (C-6); 127.49 (CH-5); 69.62 (CH₂-7); 45.80 (CH-2); 38.86 (C-Piv); 30.05 (CH₂-3); 27.21 (CH₃-Piv); 24.97 (CH₂-4); 13.82 (CH₃-2); 13.33 (CH₃-6).

**MS (FAB⁺) [m/z, (%)]**: 241.17 ([M+1]⁺, 7); 117.06 (55); 117.06 (51); 101.06 (29); 85.05 (100).

**HRMS (FAB⁺):** 241.1804 calculated for C₁₄H₂₅O₃ and 241.1506 obtained.

### 15. Preparation of (6S)-(2E,7Z,9E)-1-(tert-butylcarboxyl)-13-(3-furyl)-2,6,10-trimethyltridecan-2,7,9-triene.

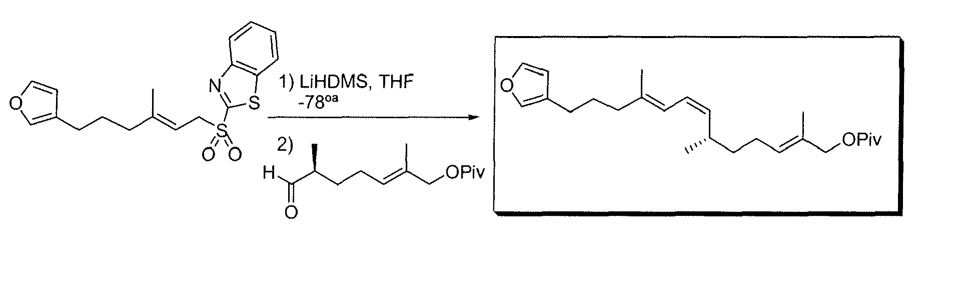

A 1.0 M solution of LiHDMS in THF (0.88 mL; 0.88 mmol) was added to a solution of the sulfone (340 mg; 0.94 µmol) in THF (2.5 mL) cooled to -78°C, the resulting intense orange solution was stirred for 1 h. Then an aldehyde solution (156 mg; 0.65 mmol) in THF (2.5 mL) was added and stirring was maintained at the same temperature for 2 h. After that time saturated NH₄Cl solution was added, the aqueous phase was extracted with TBME (3 x 30 mL) and the organic phases were washed with a saturated NaCl solution (50 mL), they were dried, filtered and vacuum-concentrated. A raw product was thus obtained which was purified by column chromatography (mobile phase: 2% AcOEt/Hexane), and which led to the diene [192 mg, 77%, **E;E / E;Z ( 1/4 )].**

Colorless liquid, Rf: 0.82 (50% AcOEt/Hexane).

**¹H-NMR (CDCl₃,** δ**):** 7.34 (1 H, s,CH-5 furyl); 7.20 (1 H, s, CH-2 furyl); 6.26 (1H, s, CH-4 furyl); 6.14 (1H, m, CH-8); 6.04 (1H, m, CH-9); 5.43 (1H, t, *J=* 7.10 Hz, CH-7); 5.11 (1H, t, *J=* 10.16 Hz, CH-3); 4.42 (2H, d, *J=* 4.32 Hz, CH₂-1); 2.61 (1H, m, CH-6); 2.40 (2H, m, CH₂-13); 2.22-1.95 (4H, m, CH₂-11, CH₂-4); 1.73 (3H, s,CH₃-2); 1.70 (2H, m, CH₂-12); 1.60 (3H, s, CH₃-10); 1.36 (2H, m, CH₂-5); 1.20 (9H, s, CH₃-^{t}Bu); 0.98 (3H, m, CH₃-6).

**¹³C-NMR (CDCl₃,** δ**):** 178.37 (C=O); 142.67 (CH-5 furyl); 138.82 (CH-2 furyl); 138.22 (C-2); 136.00 (CH-7); 135.85 (C-10); 129.09 (CH-3); 124.95 (C-3 furyl); 123.62 (CH-9); 120.39 (CH-8); 110.97 (CH-4 furyl); 69.98 (CH₂-1); 39.76 (CH₂-11); 38.85 (C-^{t}Bu); 37.16 (CH₂-5); 31.67 (CH-6); 28.18 (CH₂-12); 27.22 (CH₃-^{t}Bu); 25.72 (CH₂-13); 24.32 (CH₂-4); 21.56 (CH₃-6); 16.26 (CH₃-10); 13.76 (CH₃-2).

**MS (EI⁺) [m/z, (%)]:** 386.30 ([M]⁺, 4); 147.11 (25); 137.11 (28); 136.10 (100); 135.10 (87); 134.12 (21); 121.09 (82); 109.10 (36); 108.08 (33); 107.09 (49); 105.08 (23); 95.07 (48); 94.05 (41); 93.07 (36); 91.05 (20); 85.06 (31); 82.04 (22); 81.04 (81); 79.04 (24); 69.02 (26); 67.00 (30).

**HRMS (EI⁺):** 386.2821 calculated for C₂₅H₃₈O₃ and 386.2820 obtained.

### EXAMPLE 8:

### PREPARATION OF (6S)-(2E,7Z,9E)-13-(3-furyl)-1-hydroxy-2,6,10-trimethyltridecan-2,7,9-triene

### 1. Preparation of (6S)-(2E,7Z,9E)-13-(3-furyl)-1-hydroxy-2,6,10-trimethyltridecan-2,7,9-triene.

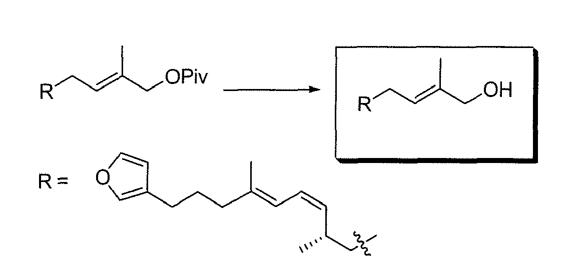

A 1.0 M solution of Dibal-H in hexane (0.56 mL; 0.56 mmol) was added to a solution of **(6*S*)-(2*E*,*7Z*,*9E*)-1-(*tert*-butylcarboxyl)-13-(3-furyl)-2,6,10-trimethyltridecan-2,7,9-triene** (73 mg; 0.19 mmol), obtained in Example 7, in CH₂Cl₂ (1.2 mL) cooled to -78°C and the resulting solution was stirred for 30 min. After that time TBME (268 µL) and H₂O (33 µL) were added to the reaction mixture and it was maintained stirring vigorously allowing it to slowly reach room temperature until the formation of a white gel, in this moment H₂O (33 µL) and 4.0 M NaOH solution (33 µL) were added and stirring was extended until the formation of a white precipitate, then Na₂SO₄ was added and the solids were vacuum filtered, the filtered liquids were evaporated to dryness obtaining a residue which was purified by column chromatography (mobile phase: 5% AcOEt/Hexane), and led to the alcohol (57 mg, 99%).

Colorless liquid, Rf: 0.60 (30% AcOEt/Hexane).

**¹H-NMR (CDCl₃, δ):** 7.37 (1H, s,CH-5 furyl); 7.23 (1H, s, CH-2 furyl); 6.29 (1H, s, CH-4 furyl); 6.18 (1 H, m, CH-8); 6.08 (1H, m, CH-9); 5.40 (1H, t, *J*= 6.68 Hz, CH-7); 5.14 (1H, t, *J*= 10.30 Hz, CH-3); 3.99 (2H, d, *J*= 5.85 Hz, CH₂-1); 2.64 (1H, m, CH-6); 2.42 (2H, m, CH₂-13); 2.26-1.97 (4H, m, CH₂-11, CH₂-4); 1.76 (3H, s,CH₃-2); 1.72 (2H, m, CH₂-12); 1.65 (3H, s, CH₃-10); 1.50 (1 H, s, -OH); 1.36 (2H, m, CH₂-5); 1.00 (3H, m, CH₃-6).

**¹³C-NMR (CDCl₃, δ):** 142.63 (CH-5 furyl); 138.79 (CH-2 furyl); 138.11 (C-2); 136.06 (CH-7); 134.64 (C-10); 126.39 (CH-3); 124.91 (C-3 furyl); 123.55 (CH-9); 120.39 (CH-8); 110.94 (CH-4 furyl); 69.00 (CH₂-1); 39.67 (CH₂-11); 37.27 (CH₂-5); 31.56 (CH-6); 28.10 (CH₂-12); 25.57 (CH₂-13); 24.32 (CH₂-4); 21.27 (CH₃-6); 16.23 (CH₃-10); 13.65 (CH₃-2).

**MS (El⁺ [m/z, (%)]:** 302.23 ([M]⁺, 3); 147.13 (21); 137.11 (27); 136.10 (77); 135.10 (100); 133.08 (22); 121.12 (36); 121.08 (60); 119.10 (21); 109.12 (30); 109.08 (23); 108.07 (25); 107.10 (58); 105.08 (35); 95.09 (43); 95.05 (67); 94.05 (60); 93.07 (61); 91.06 (37); 83.04 (21); 82.03 (25); 81.06 (78); 79.04 (36); 69.02 (44); 67.00 (43).

**HRMS (EI⁺):** 302.2246 calculated for C₂₀H₃₀O₂ and 302.2255 obtained.

### Biological Methods

### GSK-3 beta INHIBITION ASSAY

The GSK-3 beta activity of the compounds of formula (I) according to the present invention was determined by incubation of a mixture of recombinant human GSK-3 enzyme, a phosphate source and GSK-3 substrate in the presence and in the absence of the corresponding test compound, and by measuring the GSK-3 activity of this mixture. The compounds where tested at final concentrations of 25 and 50 µM.

Recombinant human glycogen synthase kinase 3 beta was assayed in MOPS 8 mM pH 7.3, EDTA 0.2 mM, MgCl₂ 10 mM and sodium orthovanadate 0.25 mM in the presence of 62.5 µM of Phospho-Glycogen Synthase Peptide-2 (GS-2), 0.5 µCi gamma-³³P-ATP and unlabelled ATP at a final concentration of 12.5 µM. The final assay volume was 20 µl. After incubation for 30 minutes at 30 °C, 15 µl aliquots were spotted onto P81 phosphocellulose papers. Filters were washed four times for at least 10 minutes each and counted with 1.5 ml of scintillation cocktail in a scintillation counter.

The compounds of formula (I) of the present invention where submitted to the above indicated assays, in order to determine both their GSK-3 inhibition activity and BACE activity inhibition. The results are indicated in Table I, in percentage of the respective enzyme activity.

| **Compound** | **% GSK-3 Activity** | |
|---|---|---|
| **(NAME)** | **25**µ**M** | **50**µ**M** |
| *tert*-Butyl-(2-furan-2-yl-ethoxy)-diphenyl-silane | - | 47,60 |
| 2-(*tert*-Butyl-diphenyl-silanyloxy)-1-furan-2-yl-ethanol | - | 48,60 |
| *tert*-Butyl-(3-furan-2-yl-propoxy)-diphenyl-silane | - | 33,48 |
| *tert*-Butyl-(5-furan-2-yl-pentyloxy)-diphenyl-silane; | | 16,13 |
| *tert*-Butyl-(6-furan-2-yl-hexyloxy)-diphenyl-silane | - | 5.08 |
| *tert*-Butyl-[3-(furan-2-ylmethylsulfanyl)-propoxy]-diphenyl-silane | | 17,39 |
| 6-Furan-3-yl-3-methyl-hex-2-enoic acid ethyl ester | 83,70 | 67,40 |
| 3-Furan-3-yl-prop-2-en-1-ol | 61,1 | 55,6 |
| *tert*-Butyl-(11-furan-3-yl-4,8-dimethyl-undeca-5,7-dienyloxy)-diphenyl-silane | 29 | 14 |
| 2,2-Dimethyl-propionic acid 13-furan-3-yl-2,6,10-trimethyl-trideca-2,7,9-trienyl ester | 45 | 27 |
| 13-Furan-3-yl-2,6,10-trimethyl-trideca-2,7,9-trien-1-ol | 83,66 | 61,18 |

## Claims

1. A compound of formula (I) 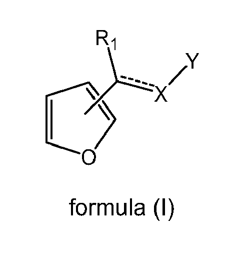 wherein
X is selected from the group consisting of -O-, -S-, -N(R^{d})-, -CH(R^{c})- and - C(R^{c})-; wherein
R^{c} is selected from the group consisting of hydrogen, substituted or unsubstituted C₁-C₃ alkyl, substituted or unsubstituted C₂-C₄ alkenyl, substituted or unsubstituted aryl, and substituted or unsubstituted aralkyl;
R^{d} is selected from the group consisting of hydrogen, substituted or unsubstituted alkyl, substituted or unsubstituted alkenyl, substituted or unsubstituted aryl, substituted or unsubstituted aralkyl, substituted or unsubstituted cycloalkyl, and substituted or unsubstituted heterocyclyl;
R₁ is selected from the group consisting of-H and -OH;
when X is -C(R^{c})-, the dotted line represents an additional bond; and
Y is selected from the group consisting of
-(CH₂)ₚW, wherein p is an integer selected from 0, 1, 2, 3, 4, 5 and 6 and W is selected from the group consisting of -OR^{a}, -SR^{a} and -NR^{a}R^{b}; and
-(CH₂)ₚ-Z-(CH₂)_{q}-C(R₂)(R₃)W, wherein p and W are as defined above; q is an integer selected from 0, 1, 2, 3 and 4; and Z is selected from the group consisting of unsubstituted alkyl, unsubstituted alkenyl, alkyl substituted with at least one C₁-C₃ alkyl group and alkenyl substituted with at least one C₁-C₃ alkyl group; and R₂ and R₃ are both hydrogen or together are =O;
wherein R^{a} and R^{b} are independently selected from the group consisting of hydrogen, protecting group, substituted or unsubstituted alkyl, substituted or unsubstituted cycloalkyl, substituted or unsubstituted alkenyl, substituted or unsubstituted aryl, substituted or unsubstituted heterocyclyl and substituted or unsubstituted aralkyl;
with the proviso that if R₁ is -OH, X is -CH(R^{c})-, R^{c} being hydrogen, Y is --(CH₂)ₚW, p being 0 and W being -OR^{a}, then R^{a} is not hydrogen;
its salts or solvates thereof.

2. Compound according to claim 1, of formula (II) 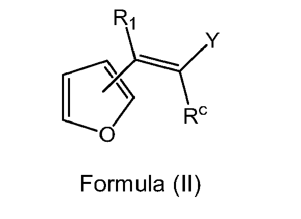 wherein R^{c}, R₁ and Y are as defined in claim 1;
its salts or solvates thereof.

3. Compound according to claim 1, of formula (III) 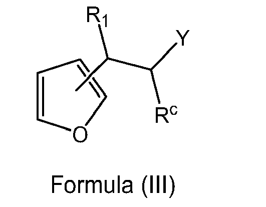 wherein R^{c}, R₁ and Y are as defined in claim 1;
its salts or solvates thereof.

4. Compound according to any of claims 1-3, wherein R₁ is hydrogen.

5. Compound according to claim 1, of formula (IV) 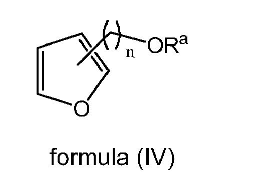 wherein R^{a} is as defined in claim 1 and n is 2, 3, 4, 5, 6, 7 or 8;
its salts or solvates thereof.

6. Compound according to claim 1, of formula (V) 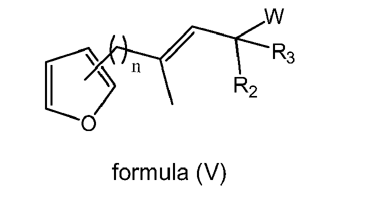 wherein W, R₂ and R₃ are as defined in claim 1 and n is 2, 3, 4, 5, 6, 7 or 8;
its salts or solvates thereof.

7. Compound according to claim 6, of formula (VI) 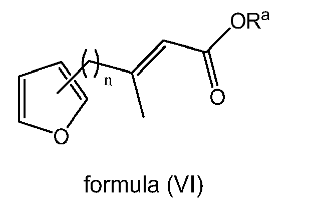 wherein R^{a} is as defined in claim 1 and n is 2, 3, 4, 5, 6, 7 or 8;
its salts or solvates thereof.

8. Compound according to claim 1, of formula (VII) 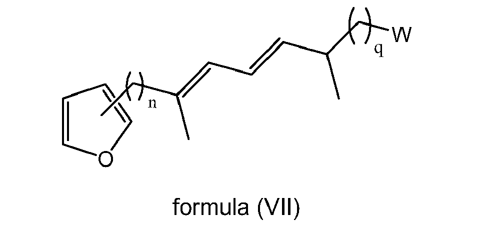
wherein W is as defined in claim 1;
n is 2, 3, 4, 5, 6, 7 or 8; and
q is 2, 3 or 4;
its salts or solvates thereof.

9. Compound according to claim 8, wherein W is -OR^{a}.

10. Compound according to claim 1, of formula (VIII) 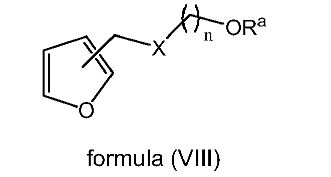 wherein X and R^{a} are as defined in claim 1, and n is 2, 3, 4, 5, 6, 7 or 8;
its salts or solvates thereof.

11. Compound according to claim 10, wherein X is -S- or -O-.

12. Compound according to claim 1, of formula IX 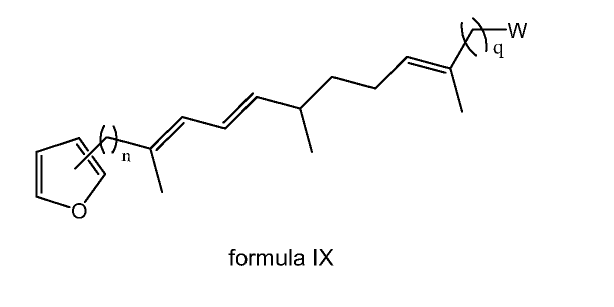
wherein W is as defined in claim 1;
n is 2, 3, 4, 5, 6, 7 or 8; and
q is 2, 3 or 4;
its salts or solvates thereof

13. Compound according to claim 1 selected from the group consisting of
- *tert*-Butyl-(2-furan-2-yl-ethoxy)-diphenyl-silane;
- 2-(tert-Butyl-diphenyl-silanyloxy)-1-furan-2-yl-ethanol;
- *tert*-Buty!-(3-furan-2-yl-propoxy)-d!phenyl-silane;
- *tert*-Butyl-(5-furan-2-yl-pentyloxy)-diphenyl-silane;
- *tert*-Butyl-(6-furan-2-yl-hexyloxy)-diphenyl-silane;
- *tert*-Butyl-[3-(furan-2-ylmethylsulfanyl)-propoxy]-diphenyl-silane
- 6-Furan-3-yl-3-methyl-hex-2-enoic acid ethyl ester;
- 3-Furan-3-yl-prop-2-en-1-ol;
- *tert*-Butyl-(11-furan-3-yl-4,8-dimethyl-undeca-5,7-dienyloxy)-diphenyl-silane;
- 2,2-Dimethyl-propionic acid 13-furan-3-yl-2,6,10-trimethyl-trideca-2,7,9-trienyl ester; and
- 13-Furan-3-yl-2,6,10-trimethyl-trideca-2,7,9-trien-1-ol;
its salts or solvates thereof.

14. Use of a compound of formula (I) as defined in claim 1, its salts or solvates thereof, in the manufacture of a medicament for the treatment and/or prevention of a GSK-3 mediated disease or condition.

15. Use according to claim 14, wherein the GSK-3 mediated disease or condition is selected from diabetes, conditions associated with diabetes, chronic neurodegenerative conditions including dementias such as Alzheimer's Disease, Parkinson's disease, progressive supranuclear palsy, subacute sclerosing panencephalitic parkinsonism, postencephalitic parkinsonism, pugilistic encephalitis, guam parkinsonism-dementia complex, Pick's disease, corticobasal degeneration, frontotemporal dementia, Huntington's Disease, AIDS associated dementia, amyotrophic lateral sclerosis, multiple sclerosis and neurotraumatic diseases such as acute stroke, epilepsy, mood disorders such as depression, schizophrenia and bipolar disorders, promotion of functional recovery post stroke, cerebral bleeding such as due to solitary cerebral amyloid angiopathy, hair loss, obesity, atherosclerotic cardiovascular disease, hypertension, polycystic ovary syndrome, syndrome X, ischaemia, brain injury, traumatic brain injury, cancer, leukopenia, Down's syndrome, Lewy body disease, inflammation, chronic inflammatory diseases, cancer and hyperproliferative diseases as hyperplasias and immunodeficiency.

16. Use according to claim 15, wherein the disease is Alzheimer's Disease.

17. Use according to claim 15, wherein the disease is type (II) diabetes.

18. Use according to claim 15, wherein the disease is depression.

19. Use according to claim 15, wherein the disease is brain injury.

20. A compound of formula I as defined in any of claims 1 to 13, its salts or solvates thereof, for use as a medicament.

21. A pharmaceutical composition comprising at least one compound of formula (I) as defined in any of claims 1 to 13, its salts or solvates thereof, and at least one pharmaceutically acceptable carrier.

22. Use of a compound of formula (I) as defined in any of claims 1 to 13, its salts or solvates thereof, as reagents for biological assays, preferably as a reactive for GSK-3 inhibition.

23. Method of treating or preventing a GSK-3 mediated disease or condition, which method comprises administering to a patient in need of such a treatment a therapeutically effective amount of at least one compound of formula (I) as defined in any of claims 1 to 13, its salts or solvates thereof, or a pharmaceutical composition thereof.

24. Process for the synthesis of a compound of formula (I) as defined in any of claims 1 to 13, its salts or solvates thereof, comprising in any order one or more of the steps selected from the group consisting of:
a) alkylation of furan with a compound of formula (XI) 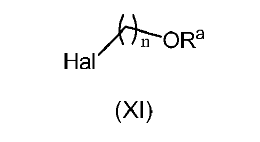 wherein R^{a} and n are as defined in claim 5 and Hal is a halogen atom;
b) Witting type reaction comprising a compound selected from the group consisting of
(i) 3-furancarboxaldehyde;
(ii) 2-furancarboxaldehyde;
(iii) a compound of formula (XII) 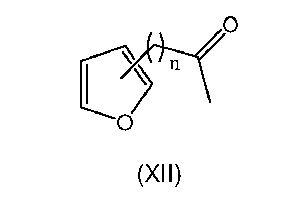 wherein n is as defined in claim 5; and
(iv) a compound of formula (XIII) 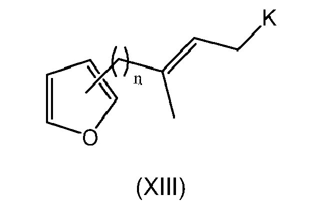 wherein K is an aldehyde group or a suphur ylide precursor; or
c) protection, alkylation or nucleophilic substitution on at least one hydroxyl group of a compound of a compound of formula (XV)
